# EUROPÄISCHE PATENTSCHRIFT

(11) **EP 3 374 347 B1**
(45) Veröffentlichungstag und Bekanntmachung des Hinweises auf die Patenterteilung: **07.08.2019**
(21) Anmeldenummer: 16794577.3
(22) Anmeldetag: 08.11.2016
(51) Int. Cl.: C07C 303/44, C07C 309/04

(54) **VERFAHREN ZUR WIEDERAUFARBEITUNG VON ALKANSULFONSÄURE**
PROCESS FOR REPROCESSING ALKANE SULFONIC ACID
PROCÉDÉ DE RETRAITEMENT D'ACIDE ALCANESULFONIQUE

(30) Priorität: 10.11.2015 EP 15193897
(43) Veröffentlichungstag der Anmeldung: 19.09.2018
(73) Patentinhaber: BASF SE, 67056 Ludwigshafen am Rhein (DE)
(72) Erfinder: SPIELMANN, Jan, 68199 Mannheim (DE); KOCH, Michael, 67346 Speyer (DE); WORTMANN, Juergen, 67117 Limburgerhof (DE); WEIGUNY, Sabine, 67251 Freinsheim (DE); RUETHER, Feelly, 67227 Frankenthal (DE); SENGPIEL, Robert, 52062 Aachen (DE)
(74) Vertreter: BASF IP Association
(86) Internationale Anmeldenummer: PCT/EP2016/076963
(87) Internationale Veröffentlichungsnummer: WO 2017/080994

(56) Entgegenhaltungen:
- EP-A2- 0 906 904
- DE-T2- 60 000 764
- US-A- 4 921 966
- US-A- 5 284 993

## Beschreibung

Die Erfindung geht aus von einem Verfahren zur Wiederaufarbeitung von Alkansulfonsäure, die in einem chemischen Prozess als Agens, Katalysator oder Lösungsmittel eingesetzt wird.

Ein Einsatzgebiet für Alkansulfonsäure, insbesondere Methansulfonsäure (MSA) ist die katalytische Alkylierung von Benzol oder substituiertem Benzol mit Olefinen zur Herstellung von linearem Alkylbenzol. Dieses ist wiederum Ausgangsstoff für die Herstellung von linearen Alkylbenzolsulfonsäuren, den am häufigsten angewandten Tensiden.

Üblicherweise werden als Katalysatoren bei der Herstellung der linearen Alkylbenzole zum Beispiel Bortrifluorid, Aluminiumtrichlorid, Schwefelsäure oder Flusssäure eingesetzt. Hierbei ist Flusssäure trotz ihrer Korrosivität, Toxizität und Flüchtigkeit, die erhebliche Instandhaltungs-, Sicherheits- und Entsorgungsprobleme beim Anlagenbetrieb mit sich bringt, der am häufigsten eingesetzte Katalysator.

Aus WO-A 2013/061336 ist bekannt, dass Methansulfonsäure als alternativer Katalysator bei der Herstellung von linearen Alkylbenzolen eingesetzt werden kann. Hierbei wird die Notwendigkeit gesehen, dass Methansulfonsäure nach ihrem Einsatz bei der Produktion der linearen Alkylbenzole nicht verworfen sondern zurückgewonnen werden soll. Problematisch ist jedoch, dass die katalytische Aktivität der rückgewonnenen Methansulfonsäure in ausreichendem Maße erhalten bleibt. Problematisch hierbei ist, dass die Methansulfonsäure während der katalysierten Reaktion Verunreinigungen aufnimmt, die die katalytische Aktivität reduzieren können. Wie eine Entfernung der Verunreinigungen erfolgen könnte, um die katalytische Aktivität zu erhalten, geht aus der WO-A 2013/061336 nicht hervor.

Neben der Herstellung von linearem Alkylbenzol können Alkansulfonsäuren auch in anderen chemischen Prozessen, insbesondere Alkylierungen und Veresterungsreaktionen als saurer Katalysator eingesetzt werden. So können zum Beispiel etablierte saure Katalysatoren wie Schwefelsäure, para-Toluolsulfonsäure oder Flusssäure weitgehend funktionsgleich ersetzt werden. Vorteil von Alkansulfonsäure, insbesondere Methansulfonsäure ist dabei die bessere katalytische Wirkung oder im Vergleich zu Flusssäure deren weit niedrigere Toxizität.

Der Einsatz von Methansulfonsäure als Katalysator bei der Alkylierung von Salicylsäuren ist beispielsweise in US 7,045,654 und die Verwendung als Katalysator bei der Alkylierung von monocyclischen Aromaten mit α-Olefinen ist aus WO-A 02/074720 bekannt. Hierbei wird jeweils beschrieben, dass die abgetrennte Methansulfonsäure enthaltende Phase durch in der Reaktion gebildete farbgebende Komponenten dunkel, beziehungsweise braun verfärbt ist.

In EP-A 323402 ist ein Verfahren zur Herstellung von flüssigen alkylierten 2-(2-Hydroxyphenyl)-benztriazol-Gemischen mit Methansulfonsäure als Katalysator beschrieben. Die als Alkylierungsmittel eingesetzten α-Olefine werden bevorzugt langsam unter die Oberflache des Reaktionsmediums zudosiert, um die Bildung von Nebenkomponenten, wie Oligomeren der α-Olefine zu reduzieren. Die Reaktion wird bevorzugt bei hohen Temperaturen von 170-180 °C durchgeführt. Die Methansulfonsäure enthaltende Phase trennt sich nach der Reaktion von der Produktphase ab. In der Reaktion entstehen gefärbte Verbindungen, die in der Produktphase und der Methansulfonsäure enthaltenden Phase zurückbleiben.

Takahashi et al., J. Jpn. Petrol. Inst. Vol. 50, No. 4, 2007, Seiten 188-194 beschreiben die durch Methansulfonsäure katalysierte Oligomerisierung von leichten Olefinen, wie Ethylen, Propylen, 1-Buten und iso-Buten zu flüssigen Kohlenwasserstoffen in einem Continous-Flow-Reaktor unter hohen Drücken von 3·10⁵ Pa bei 20 bis 40 °C. Am Ende der Reaktion werden die entstandenen flüssigen Kohlenwasserstoffe durch Phasentrennung von der als Katalysator eingesetzten Methansulfonsäure abgetrennt. Eine erhöhte Konzentration an Wasser in Methansulfonsäure hat in diesem Prozess einen negativen Einfluss auf die Ausbeute an Kohlenwasserstoffen. Die die abgetrennte Methansulfonsäure enthaltende Phase ist durch gebildete farbgebende Komponenten dunkel verfärbt.

Die oben angeführten Schriften zeigen, dass sich in durch Methansulfonsäure katalysierten Reaktionen von Olefinen schon bei sehr niedrigen Temperaturen farbgebende Verbindungen bilden können, die sich in der Kohlenwasserstoff-haltigen Produktphase und auch in der Methansulfonsäure-Phase anreichern können. Beim Wiedereinsatz der Methansulfonsäure ohne vorhergehenden Reinigungsschritt kann es dadurch zu nicht mehr tolerierbaren Gehalten an farbgebenden Komponenten in der Produktphase kommen.

Da es sich bei Alkansulfonsäuren, insbesondere Methansulfonsäure, im Vergleich zu den bisher als Katalysator eingesetzten Säuren um wesentlich wertvollere Substanzen handelt, ist für eine ökonomische Nutzung eine Rückgewinnung und Mehrfachnutzung unumgänglich. Hierbei hat sich gezeigt, dass eine einfache Rückführung ohne Aufreinigung zu einer Abnahme des Umsatzes im Reaktionssystem mit katalytischer Nutzung der Methansulfonsäure führt.

Eine mit Methansulfonsäure katalysierte Alkylierung von Aromaten mit Olefinen mit dem Ziel Olefinverunreinigungen aus aromatischen Kohlenwasserstoffen zu entfernen wurde von Tian et al., Ind. Eng. Chem. Res., Vol. 51, 2012, Seiten 13627-13631 beschrieben. Die hydrophile methansulfonsäurehaltige Phase wird nach Abtrennung ohne Reinigungsschritt als Katalysator wiederverwendet. Dabei wird mit jedem Rückführzyklus eine Abnahme der katalytischen Aktivität beobachtet. Nach dem fünften Rückführzyklus ist der Umsatz von 90% auf 66% gesunken.

Zhao et al., Catalysis Letters, Vol. 102, 2005, Seiten 219-222 beschreiben die durch Methansulfonsäure katalysierte Alkylierung von α-Methylnaphthalen mit Alkenen. Nach Phasentrennung wird die hydrophile methansulfonsäurehaltige Phase ohne Aufreinigung in den Prozess zurückgeführt. Hier zeigt sich bereits nach zweimaliger Rückführung ein Abfall des Umsatzes nach 120 Minuten von 92,8% auf 86,5%.

Neben dem Einsatz als Katalysator ist es auch möglich, die Alkansulfonsäure als Lösungsmittel in einem chemischen Produktionsprozess einzusetzen. Reaktionsgemische, die Alkansulfonsäure enthalten, sind in der Regel zweiphasig und trennen sich nach beendeter Reaktion in eine hydrophile und eine hydrophobe Phase auf. Die eingesetzte Alkansulfonsäure befindet sich dann im Wesentlichen in der hydrophilen Phase und ist mit Leichtsiedern und Schwersiedern verunreinigt. Auch hier ergibt sich die Notwendigkeit der Reinigung der Alkansulfonsäure vor Rückführung in den chemischen Prozess.

Mögliche Reinigungsverfahren für Alkansulfonsäure sind insbesondere in Verbindung mit dem Verfahren zur Herstellung von Alkansulfonsäuren beschrieben.

Aus WO-A 00/31027 ist zum Beispiel die Herstellung von Alkansulfonsäure durch Oxidation von Alkylmercaptanen, Dialkyldisulfiden oder Dialkylpolysulfiden mit Salpetersäure bekannt. Als Produkte entstehen Stickoxide, Wasser und weitere Nebenprodukte wie Schwefelsäure. Durch Oxidation mit Sauerstoff wird die Salpetersäure aus den Stickoxiden regeneriert und in den Prozess zurückgeführt. Zur Aufreinigung werden Leichtsieder und Schwersieder über eine Destillation in zwei Schritten abgetrennt und so eine reine, praktisch wasserfreie Alkansulfonsäure gewonnen. Wasser und Salpetersäure werden aus dem Rohprodukt in einer als Abtriebskolonne betriebenen Wasserabtrennkolonne bei leichtem Unterdruck abgetrennt. Das Sumpfprodukt enthält dabei 1 Gew.-% Wasser und etwa 1 Gew.-% Schwersieder, vor allem Schwefelsäure. Die Abtrennung der Schwersieder gelingt durch Destillation der Alkansulfonsäuren mit Reinheiten von mehr als 99,5 Gew.-% und Schwefelsäuregehalten von weniger als 50 ppm im hohen Vakuum, das heißt bei einem Druck von 0,1 bis 20 mbar (abs).

In WO 2015/086645 wird die Herstellung von Methansulfonsäure durch Oxidation von Dialkylsulfiden mit Stickoxiden beschrieben. Die Stickoxide werden beispielsweise mit Sauerstoff angereicherter Luft regeneriert. Danach werden die Reaktionsprodukte über zwei Destillationskolonnen von Leicht- und Schwersiedern befreit. Das so gereinigte Produkt enthält Methansulfonsäure mit einer unspezifizierten Konzentration.

In GB- A 1350328 ist die Synthese von Alkansulfonsäuren durch Chlorierung von Alkylmercaptanen oder Dialkyldisulfiden in wässriger Salzsäure beschrieben. Das Produkt der Reaktion ist eine 70 bis 85 Gew.-%ige Alkansulfonsäure. Zur Herstellung von wasserfreier Methansulfonsäure wird hier ein zweistufiger Prozess beschrieben. Im ersten Schritt wird Wasser abdestilliert und in einem zweiten Schritt die Methansulfonsäure mit einer kurzen Kolonne aus dem Sumpfprodukt herausdestilliert und über Kopf gewonnen.

CN-A 1810780 beschreibt die Synthese von Methansulfonsäure durch Reaktion von Ammoniumsulfit mit Dimethylsulfat. Dabei entstehen Ammoniummethylsulfonat und Ammoniumsulfat. Durch Zugabe von Calciumhydroxid wird lösliches Calciummethylsulfonat und unlösliches Calciumsulfat gebildet, welches leicht abgetrennt werden kann. Schwefelsäure wird zugegeben um Methansulfonsäure freizusetzen und erneut Calciumsulfat zu bilden und auszufällen. Aus der gebildeten wässrigen Lösung wird durch Destillation zuerst Wasser entfernt und dann Methansulfonsäure durch Destillation im Unterdruck gewonnen.

DE-C 197 43 901 beschreibt die Synthese von Methansulfonsäure durch Reaktion von Sulfitionen mit Dimethylsulfat. Dabei werden Sulfitionen in einem wässrigen System bei erhöhter Temperatur umgesetzt und einer starken Säure ausgesetzt. Als Nebenprodukt fällt Sulfat beispielsweise in Form von Natriumsulfat an. Die Aufreinigung der Säure erfolgt durch Destillation.

EP-A 0 675 107 beschreibt einen Prozess zu kontinuierlichen Herstellung von Alkansulfonsäurechlorid (ASC) oder Alkansulfonsäure (ASA) in dem ein Alkanmercaptan oder ein Dialkandisulfid mit Chlor in wässriger Salzsäure auch bei erhöhtem Druck umgesetzt wird. HCl und andere unter den verfahrenstechnischen Bedingungen nicht kondensierbare Leichtsieder werden nach Entspannung des Überdrucks ausgetrieben. ASC wird im bevorzugten Temperaturbereich von 10 bis 35°C hergestellt und durch eine Destillationskolonne gereinigt. ASA wird aus ASC durch Hydrolyse bei über 80°C bis 135°C mit anwesendem Wasser gewonnen. Die Reinigung von ASC und oder ASA wird beispielsweise auch mit einem Dampfstripper durchgeführt, in dem auch Reste von ASC hydrolysiert werden.

Die Entfernung von Wasser aus einer wässrigen Methansulfonsäure durch Evaporation des Wassers in einem Fallfilmverdampfer im Unterdruck ist in US 4,450,047 beschrieben. Wasser wird über Kopf abgezogen und es wird ein Produktstrom mit mehr als 99,5 Gew.-% Methansulfonsäure erhalten.

Aus US 4,938,846 ist die Entfernung von Wasser aus einer wässrigen Methansulfonsäure durch Verdampfung des Wassers in zwei hintereinandergeschalteten Fallfilmverdampfern, die beide im Unterdruck betrieben werden, bekannt.

Nachteilig bei den nach dem Stand der Technik bekannten Destillationsverfahren ist, dass der Prozess aufgrund der hohen Temperaturen und dem erforderlichen Unterdruck sehr energieaufwändig ist. Zudem können Schwersieder wie Schwefelsäure ohne besonders energieaufwendige Überführung der Alkansulfonsäure in die Gasphase nicht abgetrennt werden. Auch wird bei einigen Reinigungsprozessen die Destillationsaufgabe mit Fallfilmverdampfern gelöst, die im großindustriellen Maßstab nur schwierig einzusetzen sind.

Ein ebenfalls sehr energieintensives Verfahren ist aus US 4,035,242 bekannt, bei dem wässrige Methansulfonsäure in einem zweistufigen Destillationsprozess gereinigt wird. In der ersten Destillationskolonne wird ein Großteil des Wassers im Unterdruck als Leichtsieder entfernt. Das Methansulfonsäure enthaltende Sumpfprodukt wird in einer zweiten Rektifikationskolonne im Unterdruck verdampft und aufgetrennt, wobei die Methansulfonsäure gewonnen wird.

Die Entfernung von Schwefelsäure aus Methansulfonsäure mit Hilfe basischer Anionentauscherharze ist aus US 6,337,421 bekannt. Des Weiteren werden andere Verfahren zur Abtrennung von Schwefelsäure beschrieben, zum Beispiel Destillation oder fraktionierende Kristallisation sowie die Trennung durch Nanofiltration, die gemäß der Beschreibung der US 6,337,421 jedoch alle keine zufriedenstellenden Ergebnisse liefern.

Die Reinigung von Methansulfonsäure, die oxidierbare Verbindungen enthält, ist in EP-A 0 505 692 oder EP-A 0 373 305 beschrieben. Hierbei wird gemäß EP-A 0 505 692 Chlor zugeführt, um die Verunreinigungen in Methansulfonsäurechlorid zu überführen, das in einem weiteren Schritt zu Methansulfonsäure und Salzsäure hydrolysiert wird. Gemäß EP-A 0 373 305 wird Ozon zugeführt, durch das Methylthiosulfat in Methansulfonsäure umgewandelt wird. Diese beiden Verfahren haben jedoch den Nachteil, dass schwersiedende Komponenten wie Schwefelsäure nicht entfernt werden können und daher weitere Reinigungsschritte erforderlich sind.

Die fraktionierende Kristallisation von Methansulfonsäure und auch von Ethansulfonsäure ist aus R. A. Craig et al., J. Am. Chem. Soc., 1950, Vol. 72, Seiten 163 bis 164 oder A. Berthoud, Helv. Chim. Acta, 1929, Vol. 12, Seite 859 prinzipiell bekannt, jedoch geben die dort beschriebenen Verfahren keinen Hinweis auf eine Umsetzung in großindustriellen Verfahren.

Im Unterschied zu Verunreinigungen, die sich aus der Herstellung der Alkansulfonsäuren ergeben, kann eine in einem chemischen Prozess eingesetzte Alkansulfonsäure, insbesondere Methansulfonsäure weitergehende Verunreinigungen enthalten. Diese werden typischerweise in Leichtsieder und Schwersieder unterschieden, wobei der Siedepunkt der Leichtsieder unter dem der Alkansulfonsäure und der Siedepunkt der Schwersieder oberhalb dem der Alkansulfonsäure liegt.

Typische Leichtsieder, die in einer in chemischen Prozessen eingesetzten Alkansulfonsäure enthalten sein können, sind zum Beispiel Wasser, Salpetersäure, Chlorwasserstoff, Chlor, Alkylmercaptane, Dialkyldisulfide, Dialkylpolysulfide, Methansulfonsäuremethylester (MSA-ME), Schwefeldioxid, Ammoniak, Dimethylsulfat, Monomethylsulfat, kurzkettige Kohlenwasserstoffe, Olefine, aromatische Kohlenwasserstoffe, Benzol, kurzkettige aliphatische und aromatische Alkohole, Methylsulfonsäureester, aromatische Sulfonsäuren, Alkylsulfonsäuren, Carbonsäuren und Carbonsäureester,

Als Verunreinigungen enthaltene Schwersieder können zum Beispiel Schwefelsäure, Salze, beispielsweise Natriumsulfat, Natriumhydrogensulfat, Natriummethylsulfat, Ammoniumsulfit, Ammoniummethylsulfat, Ammoniumsulfat, Calciumhydroxid, Calciumsulfat und Calciummethylsulfat, langkettige Kohlenwasserstoffe, aromatische Kohlenwasserstoffe, Olefine, langkettige aliphatische Alkohole, aromatische Alkohole, Methylsulfonsäureester, aromatische Sulfonsäure, Alkylsulfonsäuren, Carbonsäuren oder Carbonsäureester sein.

Neben den vorstehend genannten Verunreinigungen können darüber hinaus andere auch nicht identifizierte oder nicht identifizierbare Schwersieder, insbesondere farbgebende Substanzen (Farbgeber) enthalten sein.

Aus EP-A 323402 ist ein Verfahren zur Herstellung von flüssigen alkylierten 2-(2-Hydroxyphenyl)-benztriazol-Gemischen mit Methansulfonsäure als Katalysator bekannt. In einer Ausführungsform des Verfahrens wird das Reaktionsgemisch nach beendeter Reaktion mit Wasser versetzt. Anschließend wird die Rohproduktphase von der methansulfonsäurehaltigen Phase getrennt. Aus der methansulfonsäurehaltigen Phase wird die Methansulfonsäure durch Destillation aufgearbeitet und in den Prozess zurückgeführt. Die Destillation erfolgt dabei mehrstufig. In der ersten Destillationsstufe wird Wasser entfernt und die Methansulfonsäure letztendlich über Kopf destilliert.

In der US 5,284,993 ist die Regeneration eines Alkylierungskatalysators für die Alkylierung von olefinischen Kohlenwasserstoffen mit isoparaffinen Kohlenwasserstoffen, bestehend aus starken Säuren wie Fluorsulfonsäure, und Methansulfonsäure beschrieben. Der Katalysator ist mit sogenannten säurelöslichen Ölen verunreinigt, die unerwünschte Nebenprodukte der säurekatalysierten Alkylierungsreaktion sind. Es wird beschrieben, dass eine zu hohe Konzentration dieser säurelöslichen Öle einen negativen Effekt auf die Aktivität des Katalysators und das entstehende Produkt hat. Zur Regeneration des Katalysators wird die starke Säure in einem ersten Schritt durch eine Flash-Destillation entfernt. Anschließend wird Wasser zur Mischung aus Methansulfonsäure und säurelöslichen Ölen gegeben, bis sich eine Phasentrennung einstellt. Die Phasen werden in eine hydrophobe, säurelösliche Öle enthaltende Phase und eine hydrophile Phase, die Methansulfonsäure und Wasser enthält, getrennt. Aus der hydrophilen Phase wird Wasser durch einen nicht näher beschriebenen Prozess abgetrennt und die Methansulfonsäure in den Alkylierungsprozess zurückgeführt.

Die WO-A 2013/061336 beschreibt einen Prozess zur Herstellung von linearem Alkylbenzol aus einem C₁₀-C₁₄-Alken mit einem Überschuss an Benzol, in dem Methansulfonsäure als ein Katalysator benannt ist. In der Prozessbeschreibung wird auch eine Einheit zur Katalysatorrückgewinnung genannt, ohne dass die Wirkungsweise dieser verfahrenstechnischen Stufe offenbart wurde. Die Edukte werden zusammen mit einem Alkangemisch als Lösemittel einem Alkylierungsschritt zugeführt. Das Reaktionsgemisch wird danach in einer Separationseinrichtung in Abfallströme, Rückströme und Produktströme aufgetrennt. Die Separationseinrichtung umfasst zum Beispiel zwei Phasentrenner und eine Destillationseinrichtung. Das Reaktionsgemisch ist zweiphasig und trennt sich nach beendeter Reaktion im ersten Phasentrenner in eine hydrophobe Phase und eine hydrophile Katalysatorphase auf. Die hydrophobe Phase kann durch Waschung mit Wasser oder einer Alkyllösung von Resten an Katalysator befreit werden. Anschließend wird sie destillativ aufgetrennt und das Produkt gewonnen. Das in der Destillation abgetrennte Benzol und die Alkane, sowie nicht umgesetzte Olefine, werden in den Alkylierungsschritt zurückgeführt. Verbrauchter Katalysator und Wasser werden über einen weiteren Abzug ausgetragen. Die dem ersten Phasentrenner entnommene hydrophile Methansulfonsäure enthaltende Phase wird entweder direkt in den Prozess zurückgeführt oder in eine in der WO-A 2013/061336 nicht näher beschriebene Einheit zur Katalysatorrückgewinnung eingeleitet.

Die Bildung von Dodecylbenzol aus Benzol und 1-Dodecen mit Methansulfonsäure als Katalysator in einem lösungsmittelfreien Reaktionssystem wurde von B.X. Luong et al., Journal of Catalysis, Vol. 226, 2004, Seiten 301-307, untersucht. Es konnte gezeigt werden, dass eine direkte Rückführung der methansulfonsäurehaltigen Katalysatorphase schon nach wenigen Rückführungen zu einem inakzeptablen, starken Abfall der Aktivität der Methansulfonsäure führt. Als Grund für diesen Reaktivitätsabfall wird die Anwesenheit von mehr als 0,25 Gew.-% Wasser und sich in der Katalysatorphase ansammelnden, katalytisch hemmenden, hochsiedenden Produkten angenommen. Um die katalytische Aktivität der Methansulfonsäure wieder herzustellen, wird die Methansulfonsäure enthaltende Phase mit Wasser verdünnt und mit Dichlormethan extrahiert. Anschließend wird Wasser destillativ entfernt. Das Einbringen und die nötige Entfernung von zusätzlichem Wasser und des Lösungsmittels Dichlormethan ergeben einen sehr komplexen, mehrstufigen Reinigungsprozess für die Methansulfonsäure.

Nachteil aller aus dem Stand der Technik bekannten Verfahren ist, dass diese entweder einen sehr hohen apparativen Aufwand und/oder großen Energieeinsatz erfordern, um die Verunreinigungen zu entfernen oder dass aufgrund der in der rückgeführten Alkansulfonsäure enthaltenen Verunreinigungen die katalytische Aktivität und damit der Umsatz im chemischen Prozess, der die Alkansulfonsäure als Katalysator nutzt, zurückgeht.

Darüber hinaus muss die Reaktivität der Alkansulfonsäure in Destillationsprozessen beachtet werden. Alkansulfonsäuren können mit organischen Substanzen beispielsweise Olefinen oder Aromaten auf mannigfaltige Weise reagieren. Je nach Reaktionsbedingungen und Art der organischen Reaktionspartner können dabei Zersetzungsprodukte der Alkansulfonsäure entstehen, beispielsweise der Methylester der Methansulfonsäure, Schwefeldioxid oder H₂S. Takeuchi et al., J. of the Japan Petroleum Institute, Vol. 50(4), 2007, Seiten 88-194 beschreibt die Bildung von Oligomeren von leichtflüchtigen Olefinen mit Methansulfonsäure. Mit schwerer flüchtigen aromatischen Olefinen, die auch im Prozess zur Herstellung linearer Alkylbenzole (LAB-Prozess) vorhanden sind, wird eine Reaktion der Methansulfonsäure mit den Olefinen unter Bildung dunkelgefärbter Produkte im Destillationssumpf beobachtet. Oberhalb von 150°C und bei langer Reaktionszeit, wie sie in kontinuierlichen chemischen Produktionsverfahren typisch sind, werden sogar hochviskose teerartige Produkte gebildet. Gefördert wird die Reaktion durch Anwendung einer hohen Temperatur und eine niedrige Konzentration von Wasser. Das sind aber genau die Bedingungen, die bei einer destillativen Reinigungsoperation von Alkansulfonsäure auftreten. Beispielsweise ist zum Erreichen einer ausreichenden katalytischen Aktivität der Methansulfonsäure ein Wassergehalt von weniger als 1 Gew.-% erforderlich. Um diese Konzentration destillativ erreichen zu können, sind bei moderaten Unterdrücken von etwa 0,1 bar (abs) Temperaturen von ca. 190°C erforderlich. Diese Bedingungen im Sumpf einer Destillation führen zu einer massiven Bildung dunkel gefärbter Schwersieder, die einen alleinigen destillativen Prozessschritt zur Aufreinigung von Methansulfonsäure in einem Verfahren, das Methansulfonsäure rezykliert, impraktikabel erscheinen lassen. Eine Destillation der Methansulfonsäure über Kopf erfordert noch höhere Temperaturen und/oder sehr aufwändig herzustellende niedrige Drücke. Eine solche Destillationsführung liefert zwar reine Methansulfonsäure im Kopf einer Destillationskolonne, ist aber sehr energieaufwändig, führt zu Ausbeuteverlusten und lässt stark verunreinigte hochsiedende Olefine im Sumpf der Destillation zurück.

Aufgabe der vorliegenden Erfindung ist es daher, ein Verfahren zur Wiederaufarbeitung von Alkansulfonsäure, die in einem chemischen Prozess als Agens, Katalysator oder Lösungsmittel eingesetzt wird, bereitzustellen, das mit einem geringeren apparativen Aufwand und mit geringerem Energieeinsatz durchgeführt werden kann.

Die Aufgabe wird gelöst durch ein Verfahren zur Wiederaufarbeitung von Alkansulfonsäure, die in einem chemischen Prozess als Agens, Katalysator oder Lösungsmittel eingesetzt wird, das folgende Schritte umfasst:
(a) Abtrennen eines Alkansulfonsäure enthaltenden Stromes aus einem Reaktionsgemisch, das in dem chemischen Prozess anfällt,
(b) Zufuhr des Alkansulfonsäure enthaltenden Stromes als Ausgangsschmelze in eine Schmelzekristallisation, wobei sich Kristalle der Alkansulfonsäure, von Hydraten der Alkansulfonsäure oder einer Mischung aus beiden suspendiert in Mutterlauge bilden,
(c) Durchführen einer Fest-Flüssig-Trennung, um die Kristalle aus der Mutterlauge abzutrennen,
(d) Gegebenenfalls Waschen der Kristalle, um an den Kristallen anhaftende Mutterlauge zu entfernen,
(e) Rückführen der aus der Mutterlauge abgetrennten und gegebenenfalls gewaschenen Kristalle in den chemischen Prozess.

Überraschenderweise hat sich gezeigt, dass durch die Schmelzekristallisation Verunreinigungen aus der Alkansulfonsäure in ausreichender Menge entfernt werden können, um diese wieder in dem chemischen Prozess einsetzen zu können, ohne dass der Umsatz im chemischen Prozess nennenswert zurückgeht, und zudem der notwendige Energiebedarf gegenüber den bekannten Verfahren zur Aufreinigung der Alkansulfonsäure reduziert ist.

Verunreinigungen liegen im Allgemeinen als Leichtsieder oder als Schwersieder vor. Hierbei werden unter dem Begriff "Leichtsieder" Wasser und alle Komponenten, die einen Siedepunkt unterhalb dem Siedepunkt der Alkansulfonsäure haben, verstanden. Als "Schwersieder" werden alle Komponenten bezeichnet, deren Siedepunkt oberhalb des Siedepunkts der Alkansulfonsäure liegt. Die Alkansulfonsäure ist insbesondere Methansulfonsäure.

Ein weiterer Vorteil ist, dass anders als bei derzeit eingesetzten Verfahren zur destillativen Aufreinigung der Alkansulfonsäure durch das erfindungsgemäße Verfahren insbesondere bei der Aufreinigung von Methansulfonsäure auf einfache Weise hohe Reinheiten erzielt werden können. Darüber hinaus werden durch das erfindungsgemäße Verfahren auch Schwersieder zum größten Teil aus der Alkansulfonsäure abgetrennt, was nach dem Stand der Technik nur durch energieintensive Destillation mittels Überführung der Alkansulfonsäure in den Gasraum erfolgt. Diese Überführung der Alkansulfonsäure, insbesondere Methansulfonsäure, hat jedoch den weiteren Nachteil, dass diese unter den Bedingungen bei der gasförmigen Destillation hochkorrosiv ist und den Einsatz komplexer, korrosionsgeschützter Apparate erfordert.

Um die Alkansulfonsäure rückgewinnen zu können, ist es notwendig, diese zunächst in einem ersten Schritt aus dem bei dem chemischen Prozessanfallenden Reaktionsgemisch abzutrennen. Hierzu ist es zum Beispiel möglich, Wasser zuzugeben, wobei die Alkansulfonsäure in diesem Fall in die wässrige Phase übergeht. Alternativ fällt in vielen Anwendungsfällen, zum Beispiel bei der Herstellung linearer Alkylbenzole, in denen die Alkansulfonsäure als Katalysator eingesetzt wird, bereits ein zweiphasiges Reaktionsgemisch mit einer hydrophilen und einer hydrophoben Phase an. In diesen Fällen, in denen das Reaktionsgemisch zweiphasig ist oder durch Zugabe von Wasser eine zweite Phase erzeugt wird, wird der Alkansulfonsäure enthaltende Strom vorzugsweise durch Phasentrennung aus dem Reaktionsgemisch abgetrennt. Hierzu können alle dem Fachmann bekannten Apparate zur Phasentrennung eingesetzt werden. Alternative Verfahren arbeiten zum Beispiel extraktiv oder chromatografisch. Sie haben allerdings den Nachteil geringer Selektivität oder sind sehr aufwändig.

Durch die Kristallisation werden die Verunreinigungen aus der Alkansulfonsäure entfernt und reichern sich in der Mutterlauge an. Da die Mutterlauge jedoch noch einen großen Anteil an nicht kristallisierter Alkansulfonsäure enthält, ist es bevorzugt, die Mutterlauge nach Abtrennung der Kristalle in Schritt (c) und/oder die in Schritt (b) anfallende Mutterlauge zumindest teilweise in die Schmelzekristallisation in Schritt (b) zurückzuführen.

Je nach Wassergehalt in dem der Schmelzekristallisation zugeführten Alkansulfonsäure enthaltenden Strom kristallisiert entweder die Alkansulfonsäure oder das Hydrat der Alkansulfonsäure. Da sowohl für die Kristallisation der Methansulfonsäure als auch für die Kristallisation des Hydrats der Alkansulfonsäure ein bestimmter Wassergehalt in der Schmelze erforderlich ist, kann dies erfordern, dass vor der Zugabe des Alkansulfonsäure enthaltenden Stroms in die Schmelzekristallisation in Schritt (b) der Wassergehalt eingestellt wird. Insbesondere dann, wenn die Alkansulfonsäure und nicht das Hydrat in dem chemischen Prozess eingesetzt wird, kann dies erfordern, dass vor der Kristallisation das Wasser aus dem Alkansulfonsäure enthaltenden Strom entfernt wird. Da Wasser im Vergleich zur Alkansulfonsäure ein Leichtsieder ist, ist es hierzu zum Beispiel möglich, den Alkansulfonsäure enthaltenden Strom vor Zufuhr in die Schmelzekristallisation in Schritt (b) zur Abtrennung von Leichtsiedern zu destillieren, wobei die Leichtsieder am Kopf einer Destillationskolonne abgezogen werden und ein Alkansulfonsäure, Schwersieder und Reste an Leichtsiedern enthaltender Stoffstrom am Sumpf der Destillationskolonne entnommen wird und der Alkansulfonsäure, Schwersieder und Reste an Leichtsiedern enthaltende Strom der Schmelzekristallisation in Schritt (b) zugeführt wird. Alternativ kann es bei einem zu geringen Wassergehalt auch erforderlich sein, Wasser zuzugeben. Dies ist insbesondere dann der Fall, wenn das Monohydrat der Alkansulfonsäure kristallisiert werden soll.

Hat die aus einem chemischen Prozess zurückgewonnene Alkansulfonsäure, insbesondere Methansulfonsäure einen zu hohen Wassergehalt beispielsweise mit dadurch bedingter zu niedriger katalytischer Aktivität, ist der Wassergehalt durch Leichtsiederabtrennung auf beispielsweise unter 1 Gew.-% zu senken, um eine ausreichende katalytische Aktivität zu erzielen.

Hierbei kann der Wassergehalt durch Einsatz einer Kristallisation durch zwei alternative Verfahren vorteilhaft gegenüber einer rein destillativen Leichtsiederabtrennung eingestellt werden.

Einerseits ist es möglich, durch destillative Leichtsiederabtrennung den Wassergehalt der alkansulfonsäurehaltigen Schmelze auf maximal 25 Mol-%, bezogen auf die Menge an Wasser und Alkansulfonsäure, zu senken und dann reine Alkansulfonsäure mit einem Wassergehalt unter 1 Gew.-% durch Schmelzekristallisation zu gewinnen. Dabei kann in der Destillation einerseits Energie eingespart und andererseits der Sumpf geringer thermisch belastet werden mit dem Vorteil, dass weniger zusätzliche Verunreinigungen durch Reaktion der Alkansulfonsäure mit vorhandenen Verunreinigungen gebildet werden.

Eine Leichtsiederabtrennung kann beispielsweise durch Destillation zur Darstellung reiner Methansulfonsäure dabei bei Temperaturen unter 150°C und moderaten Unterdrucken von ca. 50 bis 150 mbar geführt werden, so dass wenig Energie aufgewendet werden muss und der Wassergehalt nur bis auf 4 Gew.-% im Sumpf abgesenkt wird. Unter diesen Bedingungen kommt es nicht zu einer erheblichen Reaktion der Methansulfonsäure beispielsweise mit aromatischen Olefinen zu sehr dunkel gefärbten Produkten. Durch eine anschließende Kristallisation können sowohl das Restwasser als auch dunkel gefärbte Produkte weitgehend entfernt werden.

Dagegen führt beispielsweise eine rein destillative Senkung der Wasserkonzentration auf weniger als 1 Gew.-% bei 190°C und 100 mbar aufgrund der höheren Temperatur zur vermehrten Bildung von Nebenkomponenten.

Andererseits ist es möglich, die aus einem chemischen Prozess zurückgewonnene alkansulfonsäurehaltige Schmelze direkt zu kristallisieren und dann einer Leichtsiederabtrennung zuzuführen. Dieses Verfahren ist insbesondere dann vorteilhaft, wenn beispielsweise durch eine Leichtsiederabtrennung oder Wasserzugabe die Konzentration der methansulfonsäurehaltigen Schmelze auf 31 bis 75 Mol-%, bevorzugt von 45 bis 63 Mol-% und besonders bevorzugt von 47 bis 55 Mol-% eingestellt wird und Methansulfonsäurehydrat kristallisiert wird. Eine folgende Leichtsiederabtrennung, um eine Konzentration unter 1 Gew.-% und eine ausreichenden katalytische Aktivität zu erreichen, kann dann durchgeführt werden ohne dass sich in der Leichtsiederabtrennung zusätzliche Verunreinigungen bilden können, da vorhandene Verunreinigungen als Reaktionspartner für die Alkansulfonsäure weitgehend fehlen.

Da bei einer Kristallisation und einer nachfolgenden Fest-Flüssig-Trennung in der Regel keine vollständige Abtrennung des Produkts aus der Ausgangsschmelze möglich ist, enthält die den Kristallisator verlassende Mutterlauge noch einen großen Anteil an Produkt. Aus diesem Grund ist es bevorzugt, die an Alkansulfonsäure abgereicherte und den Kristallisator verlassende Mutterlauge vollständig oder zumindest teilweise wieder in den Wiederaufarbeitungsprozess oder auch in Reinigungsstufen des chemischen Prozess zurückzuführen. Bei Rückführung in den Wiederaufarbeitungsprozess kann die an Alkansulfonsäure abgereicherte Mutterlauge entweder zurück in den Kristallisator geleitet werden, Kristallisationskreis genannt, oder alternativ zurück in die Destillation zur Abtrennung der Leichtsieder, Destillationskreis genannt. Die Rückführung in die Destillation hat dabei den Vorteil, dass Leichtsieder, die sich aufgrund der Entfernung des Produkts in der Mutterlauge anreichern, ebenfalls entfernt werden.

Bevorzugt ist, dass die gesamte Mutterlauge, die in Schritt (c) vom Kristallisat entfernt wird oder die aus dem Kristallisator abläuft, in die Destillation zur Abtrennung von Leichtsiedern oder in die Schmelzekristallisation zurückgeführt wird.

Wenn zusätzlich das Waschen der Kristalle in Schritt (d) durchgeführt wird, fällt verunreinigte Waschflüssigkeit an, die in den Prozess zur Wiederaufarbeitung von Alkansulfonsäure rückgeführt wird. In diesem Fall ist es bevorzugt, wenn die Waschflüssigkeit mit der Mutterlauge zusammengeführt wird.

Da sich in der Mutterlauge durch das Auskristallisieren der Alkansulfonsäure insbesondere bei Rückführung in die Destillation oder die Schmelzekristallisation Schwersieder anreichern, ist es weiterhin bevorzugt, wenn die in Schritt (c) entfernte Mutterlauge zumindest teilweise einer Schwersiederabtrennung zugeführt wird, um Schwersieder aus der Mutterlauge zu entfernen. Nach Abtrennung der Schwersieder wird die Mutterlauge in den Prozess zur Wiederaufarbeitung von Alkansulfonsäure zurückgeführt. Die Zufuhr der Mutterlauge in die Schwersiederabtrennung und anschließende Rückführung in den Prozess zur Wiederaufarbeitung von Alkansulfonsäure wird Schwersiederkreis genannt. Die Schwersiederabtrennung erfolgt dabei vorzugsweise vor der Einleitung der Mutterlauge in die Destillation zur Abtrennung der Leichtsieder oder in die Schmelzekristallisation. Besonders bevorzugt ist es dabei, wenn die Mutterlauge nach dem Entfernen der Schwersieder in die Destillation zur Abtrennung der Leichtsieder zurückgeführt wird. Dies erlaubt es, dass noch in der Mutterlauge als Verunreinigungen enthaltene Leichtsieder in der Destillation ebenfalls aus der Mutterlauge entfernt werden. Alternativ ist es auch möglich, die Mutterlauge nach dem Entfernen der Schwersieder teilweise zu kondensieren und den kondensierten Anteil in die Destillation zur Abtrennung der Leichtsieder zurückzuführen und den nicht kondensierten Anteil als Leichtsieder aus dem Prozess auszuschleusen. Durch die Teilkondensation der Mutterlauge und das Ausschleusen des nicht kondensierten Anteils an Leichtsiedern wird bei einer Rückführung des kondensierten Anteils der Mutterlauge in die Destillation zur Abtrennung der Leichtsieder der in der Destillation zu entfernende Anteil an Leichtsiedern reduziert, so dass die Destillation insgesamt für einen geringeren Durchsatz auszulegen ist, wodurch wiederum Investitionskosten und insbesondere Energie eingespart werden können.

Die Schwersiederabtrennung kann beispielsweise durch eine Verdampfung realisiert werden. Hierbei verdampfen die in der Mutterlauge enthaltenen Leichtsieder und die Alkansulfonsäure und die Schwersieder verbleiben in der Flüssigphase. Um die Alkansulfonsäure nicht zu schädigen und die Verdampfung bei möglichst niedrigen Temperaturen durchführen zu können, wird die Verdampfung vorzugsweise bei einem Druck unterhalb des Umgebungsdrucks durchgeführt. Die bei der Verdampfung entstehenden, Alkansulfonsäure enthaltenden Brüden können kondensiert und als Flüssigkeit zurück in die Destillation zur Abtrennung von Leichtsiedern oder in die Schmelzekristallisation in Schritt (b) oder, besonders bevorzugt, in den chemischen Prozess geleitet werden. Durch die Kondensation der Brüden besteht auch die Möglichkeit, Leichtsieder zu entfernen. In diesem Fall wird die Kondensation so durchgeführt, dass die Alkansulfonsäure kondensiert, die Leichtsieder jedoch in der Gasphase verbleiben. In einem Gas-Flüssig-Phasentrenner lassen sich dann die die Alkansulfonsäure enthaltende Flüssigphase und die die Leichtsieder enthaltende Gasphase voneinander trennen und die in der Gasphase enthaltenen Leichtsieder können aus dem Prozess ausgeschleust werden. Eine Verdampfung kann auch im Sinne einer Strippung durch ein Schleppgas gefördert werden.

Die bei der Verdampfung anfallende Schwersieder-enthaltende Fraktion enthält im Allgemeinen auch noch einen Anteil an Alkansulfonsäure, die gegebenenfalls in einen nachfolgenden Reinigungsschritt zurückgewonnen werden kann.

Sowohl die Destillation zur Abtrennung von Leichtsiedern als auch die Verdampfung der Alkansulfonsäure zur Entfernung von Schwersiedern werden bei einem Druck unterhalb des Umgebungsdrucks durchgeführt. Bevorzugt werden die Destillation zur Abtrennung von Leichtsiedern und die Verdampfung zur Entfernung von Schwersiedern bei einem Druck im Bereich 5 bis 500 mbar (abs), besonders bevorzugt von 10 bis 100 mbar (abs) durchgeführt. Dies erlaubt eine für die Alkansulfonsäure produktschonende Destillation beziehungsweise Verdampfung. Bei höheren Drücken wären die notwendigen Temperaturen für die Destillation beziehungsweise Verdampfung so hoch, dass eine Produktschädigung, insbesondere eine Zersetzung der Alkansulfonsäure nicht ausgeschlossen werden kann. Es ist bekannt, dass Destillationsverfahren unter Einsatz von Schleppmitteln, sogenannten Strippverfahren, bei höheren Drücken durchgeführt werden können. Diese Prozessführung wird im Rahmen der Erfindung als äquivalent zur Anwendung eines Unterdrucks angesehen.

Wenn die Alkansulfonsäure Methansulfonsäure ist und reine Methansulfonsäure kristallisiert werden soll, ist die Destillation zur Abtrennung von Leichtsiedern so durchzuführen, dass die Ausgangsschmelze eine Konzentration von mindestens 76 Mol.-%, bevorzugt mindestens 82 Mol.-% und besonders bevorzugt mindestens 90 Mol.-% aufweist. Die Druck- und Temperatureinstellungen der Destillation zur Abtrennung von Leichtsiedern können dabei in weiten Bereichen variiert werden, sind aber durch die stoffspezifischen Dampfdruckkurven miteinander verbunden. Der besonders bevorzugte Wert der Methansulfonsäurekonzentration in einem Methansulfonsäure-Wassergemisch von 90 Mol.-% ist in einem bevorzugten Druckbereich von 40 bis 130 mbar und den korrespondierenden Sumpftemperaturbereichen von 160°C bis 200°C zu erreichen. Der angegebene Temperaturbereich ist um typischerweise 10 bis 20 K höher als physikalisch stoffspezifisch vorgegeben, da durch Strömung in der Kolonne Druckverluste und durch Isolationsschwächen Wärmeverluste auftreten können.

Wenn die Alkansulfonsäure Methansulfonsäure ist und das Methansulfonsäurehydrat kristallisiert werden soll, ist die Destillation zur Abtrennung von Leichtsiedern so durchzuführen, dass die Ausgangsschmelze eine Konzentration von 31 bis 75 Mol.-%, bevorzugt von 45 bis 63 Mol.-% und besonders bevorzugt von 47 bis 55 Mol.-% aufweist. Die Druck- und Temperatureinstellungen der Destillation zur Abtrennung von Leichtsiedern können auch hier in weiten Bereichen variiert werden. Ein besonders bevorzugter Wert der Methansulfonsäurekonzentration von 51 Mol.-% ist beispielsweise bei einem Druck von 40 bis 130 mbar und einer Sumpftemperatur von 80 bis 120°C zu erreichen.

Wenn die Alkansulfonsäure Ethansulfonsäure ist und reine Ethansulfonsäure kristallisiert werden soll, ist die Destillation zur Abtrennung von Leichtsiedern so durchzuführen, dass die Ausgangsschmelze eine Konzentration von mindestens 76 Mol.-%, bevorzugt mindestens 82 Mol.-% und besonders bevorzugt mindestens 90 Mol.-% aufweist. Die Druck- und Temperatureinstellungen der Destillation zur Abtrennung von Leichtsiedern können dabei in wie bei der Herstellung von Methansulfonsäure in weiten Bereichen variiert werden. Da Ethansulfonsäure einen deutlich niedrigeren Dampfdruck als Methansulfonsäure besitzt, liegen die bevorzugten Destillationsdrücke entsprechend tiefer. Eine alternative Erhöhung der Destillationstemperaturen weit über 200°C hinaus ist aufgrund merkbarer Ethansulfonsäure-Zersetzung schwierig.

Wenn die Alkansulfonsäure Ethansulfonsäure ist und das Ethansulfonsäurehydrat kristallisiert werden soll, ist die Destillation zur Abtrennung von Leichtsiedern so durchzuführen, dass die Ausgangsschmelze eine Konzentration von 31 bis 75 Mol.-%, bevorzugt von 45 bis 63 Mol.-% und besonders bevorzugt von 47 bis 55 Gew.-% aufweist.

Die Destillation kann dabei in jeder beliebigen, dem Fachmann bekannten Destillationseinrichtung durchgeführt werden. Üblicherweise wird die Destillation in einer Destillationskolonne durchgeführt, die Einbauten enthalten kann. Übliche Einbauten sind zum Beispiel Böden oder strukturierte oder unstrukturierte Packungen. Als Böden können alle bekannten Böden, zum Beispiel Siebböden, Glockenböden, Tunnelböden oder Ventilböden, eingesetzt werden. Strukturierte Packungen können beispielsweise solche aus Keramik oder Kunststoffen wie PFTE oder PFA sein. Unstrukturierte Packungen sind zum Beispiel Füllkörperpackungen, wobei alle gängigen Füllkörper verwendet werden können, z.B. solche aus Keramik, Kunstoffen wie PFTE oder PFA.

Im Allgemeinen wird der Alkansulfonsäure enthaltende Strom nahe am Kopf der Destillationskolonne aufgegeben. Über Kopf werden die Leichtsieder abgetrennt und einer Aufarbeitung oder einer Entsorgung zugeführt. Am Sumpf der Destillationskolonne wird ein Alkansulfonsäure, Schwersieder und Reste an Leichtsiedern, insbesondere Wasser, enthaltender Stoffstrom entnommen und der Schmelzekristallisation als Ausgangsschmelze zugeführt. Hierbei ist die Ausgangsschmelze im Allgemeinen einphasig flüssig. Das heißt, dass auch die Alkansulfonsäure vollständig in der flüssigen Phase enthalten ist.

Da die Destillation und die Schmelzekristallisation bei unterschiedlichen Temperaturen durchgeführt werden, ist es unabhängig von der eingesetzten Destillationseinrichtung notwendig, den Alkansulfonsäure enthaltenden Stoffstrom abzukühlen, bevor dieser der Schmelzekristallisation zugeführt wird. Selbst bei den beispielhaft angegebenen Unterdrücken der Leichtsiederabtrennung ist es notwendig, die Destillation bei Wärmezufuhr durchzuführen, um die Sumpftemperatur im Fall der Kristallisation reiner Methansulfonsäure im Bereich von 160 bis 191°C und im Falle der Kristallisation von Methansulfonsäurehydrat im Bereich von 86 bis 112°C einzustellen. Da die Schmelztemperatur eines Methansulfonsäure-Wassergemisches abhängig vom Wassergehalt im Bereich von -54 bis +20°C liegt, muss zunächst eine entsprechende Abkühlung des Sumpfaustrages bevorzugt bis auf eine Temperatur knapp oberhalb der Schmelztemperatur der Ausgangsschmelze erfolgen. Alternativ ist es auch möglich, die Schmelze vor dem Eintritt in den Kristallisator zu unterkühlen. Eine solche Fahrweise ist allerdings nicht bevorzugt, da eine ungewollte Kristallisation in einem Wärmetauscher schwer auszuschließen ist. Wenn eine andere Alkansulfonsäure aufgereinigt werden soll, müssen die Temperaturen entsprechend an die Siedetemperatur beziehungsweise Schmelztemperatur der Alkansulfonsäure angepasst werden.

Durch die Destillation zur Abtrennung von Leichtsiedern wird der Alkansulfonsäure enthaltende Strom vorzugsweise so weit von Leichtsiedern befreit, dass der Anteil an Verunreinigungen in dem der Schmelzekristallisation als Ausgangsschmelze zugeführten Alkansulfonsäure, Schwersieder und Reste an Leichtsiedern enthaltenden Stoffstrom ohne Berücksichtigung von enthaltenem Wasser maximal 6 Gew.-%, bevorzugt maximal 3 Gew.-% in der Ausgangsschmelze beträgt. Besonders bevorzugt ist es, wenn der Anteil an Verunreinigungen ohne Berücksichtigung von Wasser kleiner als 2 Gew.-% ist.

Es handelt sich bei den Angaben um nur typische Werte, die darüber hinaus abhängig vom chemischen Prozess, in dem die Alkansulfonsäure eingesetzt wird, und dem Schwersiedergehalt sind.

Im Unterschied zur Destillation, die bei einem Druck unterhalb des Umgebungsdrucks durchgeführt wird, erfolgt die Schmelzekristallisation im Allgemeinen bei Umgebungsdruck. Im Falle der reinen Methansulfonsäure wird die Schmelzekristallisation dabei vorzugsweise im Bereich von - -10°C bis 19°C durchgeführt.

Um in der Schmelzekristallisation reine Methansulfonsäure zu gewinnen, das heißt, Methansulfonsäure mit einem Anteil an Verunreinigungen und Wasser von weniger als 1 Gew.-%, bevorzugt von weniger als 0,5 Gew.-% und insbesondere von weniger als 0,2 Gew.-%, wird der Schmelzekristallisation ein Alkansulfonsäure, Schwersieder und Reste an Leichtsiedern enthaltender Stoffstrom zugeführt, der mindestens 76 Mol.-%, bevorzugt mindestens 82 Mol.-% und insbesondere bevorzugt mindestens 90 Mol.-% Methansulfonsäure bezogen auf die Gesamtmenge an Methansulfonsäure und Wasser im Alkansulfonsäure, Schwersieder und Reste an Leichtsiedern enthaltenden Stoffstrom enthält.

Weiterhin enthält der Alkansulfonsäure, Schwersieder und Reste an Leichtsiedern enthaltende Stoffstrom vor der Einspeisung von Rückführungen maximal 6 Gew.-% Verunreinigungen, vorzugsweise maximal 3 Gew.-% und insbesondere maximal 2 Gew.-% bezogen auf die Gesamtmasse des Alkansulfonsäure, Schwersieder und Reste an Leichtsiedern enthaltenden Stoffstroms. Liegen im Stoffgemisch neben Wasser und Alkansulfonsäure Verunreinigungen vor, ändern sich die molaren zur Kristallisation geeigneten Konzentrationsverhältnisse zwischen Wasser und Alkansulfonsäure nicht wesentlich. Durch Rückführung im Schwersiederkreis können die Konzentrationen an Verunreinigungen, beispielsweise an Schwefelsäure, über die angegebenen Werte hinaus steigen und dabei den Anteil an Wasser verringern. Liegen höhere Konzentrationen an Verunreinigungen vor, sind tiefere Kristallisationstemperaturen möglich und erforderlich.

Zur Gewinnung der reinen Methansulfonsäure wird die Schmelzekristallisation bei einer Temperatur im Bereich von -50 bis 19°C und bevorzugt im Bereich von -10 bis 19°C, weiter bevorzugt bei einer Temperatur im Bereich von -2 bis 18°C und insbesondere bei einer Temperatur im Bereich von 6 bis 16°C durchgeführt. Hohe Kristallisationstemperaturen sind bevorzugt, da hierdurch der Energieaufwand bei der Kristallisation geringer ist als bei niedrigeren Kristallisationstemperaturen.

Zur Gewinnung des Monohydrats der Methansulfonsäure in der Schmelzekristallisation enthält der der Schmelzekristallisation als Ausgangsschmelze zugeführte Alkansulfonsäure, Schwersieder und Reste an Leichtsiedern enthaltende Stoffstrom vorzugsweise 31 bis 75 Mol.-% Methansulfonsäure, bevorzugt 45 bis 63 Mol.-%, und besonders bevorzugt 47 bis 55 Mol-% und insbesondere 48 bis 52 Mol.-% Methansulfonsäure jeweils bezogen auf die Gesamtmenge an Wasser und Methansulfonsäure im Alkansulfonsäure, Schwersieder und Reste an Leichtsiedern enthaltenden Stoffstrom. Auch hier liegt der Anteil an Verunreinigungen vorzugsweise bei maximal 6 Gew.-%, weiter bevorzugt bei maximal 3 Gew.-% und insbesondere bei maximal 2 Gew.-% bezogen auf die Gesamtmasse des Alkansulfonsäure, Schwersieder und Reste an Leichtsiedern enthaltenden Stoffstroms. Liegen in Stoffgemisch neben Wasser und Alkansulfonsäure Verunreinigungen vor, ändern sich die molaren zur Kristallisation geeigneten Konzentrationsverhältnisse zwischen Wasser und Alkansulfonsäure nicht wesentlich. Es handelt sich bei den Angaben um nur typische Werte, die darüber hinaus abhängig vom MSA-Herstellverfahren und dem Schwersiedergehalt sind. Liegen hohe Konzentrationen an Verunreinigungen vor, sind tiefere Kristallisationstemperaturen möglich und erforderlich.

Die Temperatur, bei der die Schmelzekristallisation des Monohydrats der Methansulfonsäure durchgeführt wird, liegt dabei im Bereich von -50 bis 12°C und bevorzugt im Bereich von -15 bis 12°C, weiter bevorzugt im Bereich von -8 bis 12°C und insbesondere im Bereich von 0 bis 12°C. Hohe Kristallisationstemperaturen sind bevorzugt, da sie einen niedrigeren Energieaufwand bei der Kristallisation benötigen.

Je nach Art und Konzentration der Verunreinigungen können die optimalen Kristallisationsbedingungen unterschiedlich sein. Diese sollten entsprechend zum Beispiel durch Versuche angepasst werden. Bei kleinen Konzentrationen an Verunreinigungen liegen die Kristallisationsbedingungen sehr nahe an denen des reinen Zweistoffgemischs aus Wasser und Methansulfonsäure.

Es hat sich gezeigt, dass sowohl bei der Herstellung der reinen Methansulfonsäure als auch bei der Herstellung des Hydrats der Methansulfonsäure jeweils ein Anteil von 6 Gew.-% bezogen auf die Gesamtmasse des Alkansulfonsäure, Schwersieder und Reste an Leichtsiedern enthaltenden Stoffstromes Schwefelsäure unkritisch ist und die Kristallisationsbedingungen nur gering beeinflusst. Beispielsweise zeigt sich, dass eine Verunreinigung von 4 Gew.-% Schwefelsäure in einem Gemisch aus Wasser und Methansulfonsäure die Kristallisationstemperatur von Methansulfonsäure um ca. 3°C absenkt. Beispielsweise zeigt sich auch, dass eine Verunreinigung von 4 Gew.-% Schwefelsäure in einem Gemisch aus Wasser und Methansulfonsäure die Kristallisationstemperatur von Methansulfonsäurehydrat um ca. 2°C absenkt.

Als Kristallisator, in dem die Schmelzekristallisation durchgeführt wird, kann jeder für eine Kristallisation geeignete Apparat eingesetzt werden. Die Wärme kann im Kristallisator zum Beispiel durch Mantelkühlung oder durch geeignete Einbauten, beispielsweise von einem Kältemittel durchströmte Rohrleitungen im Kristallisator entzogen werden, bis eine für die Kristallisation ausreichend niedrige Temperatur erreicht ist. Als Kältemittel, das bei der Mantelkühlung durch eine Doppelwandung des Kristallisators strömt oder das in der durchströmten Rohrleitung eingesetzt wird, eignet sich zum Beispiel ein Gemisch aus Wasser und Ethylenglykol. Alternativ ist es auch möglich, eine direkte Kühlung durch ein verdampfendes Kältemittel, beispielsweise Kohlenstoffdioxid, durchzuführen.

Im Kristallisator wird die Ausgangschmelze in einer Ausführungsform, dem Suspensionskristallisationsverfahren, durch Abkühlung in eine Alkansulfonsäure-Kristalle enthaltende Suspension überführt. Hierzu können Feststoffkristalle aus der Alkansulfonsäure direkt in der Schmelze wachsen und so die Suspension bilden oder alternativ können sich die Feststoffkristalle als Schicht auf einer gekühlten Wand abscheiden, von der sie anschließend abgekratzt und in der Mutterlauge resuspendiert werden. Als Apparate eignen sich zum Beispiel Rührkessel, Kratzkühler oder Scheibenkristallisatoren.

In einer alternativen Ausführungsform wird eine Schichtkristallisation durchgeführt. Hierbei bildet sich das Kristallisat als zusammenhängende anhaftende Schicht an einer gekühlten Oberfläche des Kristallisators. Die Fest/Flüssig-Trennung erfolgt in diesem Fall durch Abfließen der Mutterlauge in einem Schwerefeld. Die Schichtkristallisation kann sowohl als statische als auch als dynamische Schichtkristallisation durchgeführt werden.

Bei der statischen Schichtkristallisation wird die Ausgangsschmelze in einen geeigneten Wärmetauscher eingefüllt, beispielsweise einen Rohrbündelwärmetauscher oder einen Plattenwärmetauscher, und durch langsame Temperaturabsenkung abgekühlt, wodurch die Ausgangsschmelze teilweise erstarrt. In einem weiteren Schritt wird die Mutterlauge abgelassen und die Temperatur wieder erhöht. Hierdurch wird zunächst eine stark verunreinigte Fraktion aus der Kristallschicht abgeschmolzen, bevor das Produkt mit hoher Reinheit abgeschmolzen wird. Nachteil des statischen Kristallisationsverfahrens ist jedoch die üblicherweise geringe Raum-Zeit-Ausbeute, da der Wärme- und Stofftransport zu den Abscheideflächen nur durch freie Konvektion erfolgt. Im Unterschied dazu wird bei der dynamischen Schichtkristallisation eine erzwungene Konvektion durch Umpumpen der Mutterlauge durch volldurchströmte Rohre, durch Aufgabe als Rieselfilm oder durch Einleiten von Inertgas in ein mit Mutterlauge gefülltes Rohr oder durch Pulsieren erzeugt.

Bei der Suspensionskristallisation wird dem Kristallisator eine Suspension entnommen, bei der die Kristalle in der Mutterlauge suspendiert sind. Da aus der Ausgangsschmelze Alkansulfonsäure kristallisiert ist, ist der Anteil der geschmolzenen Alkansulfonsäure in der dem Kristallisator entnommenen Mutterlauge geringer als in der dem Kristallisator zugeführten Ausgangsschmelze. Auch ist die Konzentration an Verunreinigungen in der Mutterlauge höher, da diese weitgehend nicht kristallisieren. Als Mutterlauge wird nur der flüssige Anteil, das heißt die flüssige Phase der Suspension, bezeichnet.

Um die Mutterlauge und an den Kristallen anhaftende Verunreinigungen zu entfernen, ist es möglich und bevorzugt, die Kristalle in Schritt (d) zu waschen. Hierzu werden die Kristalle mit einer Waschflüssigkeit in Kontakt gebracht, mit der die Verunreinigungen entfernt werden.

Zum Waschen der Kristalle im Schritt (d) kann jeder geeignete Wäscher genutzt werden. Hierbei ist es möglich, einen separaten Wäscher einzusetzen oder die Fest-Flüssig-Trennung und Waschung in einem Apparat durchzuführen. Ein dazu geeigneter Apparat ist zum Beispiel eine Waschkolonne. In der Waschkolonne werden die zu reinigenden Kristalle und die Waschflüssigkeit vorzugsweise im Gegenstrom geführt. Da Alkansulfonsäuren, insbesondere Methansulfonsäure, korrosiv ist, ist es notwendig, neben den Produktionsapparaten auch den Kristallisator, den Apparat zur Fest-Flüssig-Trennung und den Wäscher so auszustatten, dass dieser verfahrenstechnisch beständig ist. Insbesondere muss vermieden werden, dass die Alkansulfonsäure durch korrodierte und sich ablösende Bestandteile des Apparats verschmutzt wird. Geeignete korrosionsstabile Materialien, aus denen der Wäscher gefertigt werden kann, sind zum Beispiel Gläser, korrosionsfeste Stähle, emaillierte Stähle oder Kunststoffe. Die Kunststoffe können dabei sowohl als Auskleidungsmaterialien als auch tragend zum Einsatz kommen. Ein geeigneter Kunststoff ist zum Beispiel Polyethylen hoher Dichte oder auch PTFE. Kunststoffe sind vor allem als Konstruktionsmaterial oder zur korrosiven Isolierung des Apparateaußenmantels geeignet. Einige Apparateteile können möglicherweise für Kunststoffe zu hoch mechanisch belastet sein. Dann kann eine Konstruktion so erfolgen, dass die belasteten Anlagenteile beispielsweise auch aus mechanisch stabilem, emailliertem Stahl gefertigt werden.

Als Waschflüssigkeit können zum Beispiel Wasser, wässrige Alkansulfonsäure, Schwefelsäure oder andere Lösemittel eingesetzt werden. Diese haben jedoch alle den Nachteil, dass die Kristalle aus der Alkansulfonsäure gelöst werden können. Zudem können zusätzlich Verunreinigungen eingebracht werden. Anstelle der vorstehend genannten Waschflüssigkeiten ist es daher bevorzugt, geschmolzenes Kristallisat als Waschlösung einzusetzen. Mit dem geschmolzenen Kristallisat werden die an den Kristallen anhaftende Mutterlauge und die Verunreinigungen entfernt. Da das als Waschflüssigkeit eingesetzte geschmolzene Kristallisat durch die Mutterlauge und durch die Verunreinigungen, die von den Kristallen abgewaschen werden, verunreinigt wird und die Waschflüssigkeit aufgrund ihrer Zusammensetzung einen hohen Anteil an Wertprodukt enthält, ist es bevorzugt, das als Waschflüssigkeit eingesetzte geschmolzene Kristallisat nach dem Waschen der Kristalle in die Destillation zur Abtrennung von Leichtsiedern oder in die Schmelzekristallisation zurückzuführen.

Wenn geschmolzenes Kristallisat als Waschflüssigkeit genutzt wird, kristallisiert im Allgemeinen auch ein Teil der Waschflüssigkeit an den zu reinigenden Kristallen aus.

Um ein Sedimentieren der Kristalle aus der Suspension beim Transport zwischen den einzelnen Apparaten, insbesondere zwischen dem Kristallisator und dem Wäscher zu verhindern, ist es bevorzugt, die Suspension zu homogenisieren. Hierzu können zum Beispiel Rührer oder Pumpen eingesetzt werden. Dem Wäscher kann entweder direkt die dem Kristallisator entnommene Suspension zugeführt werden oder alternativ ist es auch möglich, die Suspension vor der Zugabe in den Wäscher aufzubereiten. Hierzu werden die in der Mutterlauge suspendierten Kristalle zunächst mechanisch abgetrennt. Hierzu kann jedes bekannte Trennverfahren für Fest/Flüssig-Trennungen eingesetzt werden. Geeignete Trennverfahren sind zum Beispiel Sieben, Pressen, Filtration, Zentrifugation, Sedimentation und Dekantieren. Nach dem Abtrennen der Mutterlauge werden die Kristalle in der Waschflüssigkeit resuspendiert und die Suspension in den Wäscher eingeleitet.

Wenn geschmolzenes Kristallisat als Waschflüssigkeit eingesetzt wird, ist es bevorzugt, wenn die Temperatur so gewählt wird, dass das geschmolzene Kristallisat zum Waschen der Kristalle eine Temperatur aufweist, die 0,1 bis 15°C oberhalb der Erstarrungstemperatur der Alkansulfonsäure liegt. Bevorzugt liegt die Temperatur als Waschflüssigkeit eingesetzten Kristallisats 1 bis 10°C oberhalb der Erstarrungstemperatur der Alkansulfonsäure und insbesondere 2 bis 4°C oberhalb der Erstarrungstemperatur der Alkansulfonsäure.

Der Wäscher wird dabei vorzugsweise so betrieben, dass die Verweilzeit der zu waschenden Kristalle in dem Wäscher im Bereich von 1 bis 25 min und bevorzugt im Bereich von 1 bis 15 min liegt. Insbesondere, aber nicht ausschließlich bei Gegenstromführung von Kristalle enthaltender Suspension und geschmolzenem Kristallisat als Waschflüssigkeit hat sich allerdings gezeigt, dass bereits mit einer Verweilzeit von 2 bis 8 min eine ausreichende Reinigungswirkung erzielt wird.

Um die Reinigungswirkung zu verbessern, ist es möglich, die Kristalle mehrfach zu waschen. Hierzu kann das Waschen in Schritt (d) oder auch die Sequenz aus Kristallisation in Schritt (b), die Fest-Flüssig-Trennung in Schritt (c) und das Waschen in Schritt (d) mehrfach durchlaufen werden oder auch mit einer Teilrückführung betrieben werden. Bevorzugt ist jedoch ein einmaliger Durchlauf durch Kristallisation und Waschung. Bei geringen Ansprüchen an die Produktreinheit kann auch auf das Waschen der Kristalle verzichtet werden.

Die drei genannten Stoffstromkreise Destillationskreis, Kristallisationskreis und Schwersiederkreis führen über Anlagenbereiche, die auf teilweise sehr unterschiedlichen Temperaturniveaus liegen. Um die in das Verfahren eingebrachte Energie gut zu nutzen und weiterhin die Energiemenge, die zum Erwärmen und Abkühlen der Stoffströme benötigt wird, möglichst gering zu halten, ist es bevorzugt, wenn die Stoffstromkreise über Wärmeübertrager geführt werden, die im Gegenstrom Wärme übertragen. Beispielsweise wird der aus dem Sumpfaustrag der Leichtsiederdestillation entnommene, Alkansulfonsäure, Schwersieder und Reste an Leichtsiedern und Schwersiedern enthaltende Stoffstrom vor Zugabe in die Schmelzekristallisation abgekühlt und im Gegenzug die an Alkansulfonsäure abgereicherte Mutterlauge, die in die Destillation zurückgeführt wird, aufgeheizt. Dabei ist es besonders bevorzugt, wenn Wärme von dem abzukühlenden, Alkansulfonsäure, Schwersieder und Reste an Leichtsiedern und Schwersiedern enthaltenden Stoffstrom an die zu heizende und an Alkansulfonsäure abgereicherte Mutterlauge übertragen wird.

Wenn effiziente Kristallisations- und Waschverfahren zur Verfügung stehen, ist es möglich, im Schwersiederkreis, das heißt der im Kreis gefahrenen Mutterlauge, die Leichtsieder und Schwersieder zu hohen Konzentrationen aufzupegeln, ohne dass die kristallisierten Alkansulfonsäuren marktgerechte Reinheitsspezifikationen verfehlen. Dabei können in der Ausgangsschmelze Verunreinigungen an Leichtsiedern und Schwersiedern in Summe von bis zu 8 Gew.-% unschädlich toleriert werden. Hierdurch können die über hohe Temperaturdifferenzen geführten Strommengen im Schwersiederkreis so klein gehalten werden, dass deren Erwärmungs- und Abkühlaufwand klein ist gegenüber dem ohnehin verfahrenstechnisch notwendigen Wärmebedarf. Das erfindungsgemäße Verfahren benötigt somit weniger Energie als die herkömmlichen Verfahren.

Der chemische Prozess, in dem die Alkansulfonsäure eingesetzt wird, ist zum Beispiel eine Alkylierung oder eine Veresterungsreaktion. Neben diesen beiden Reaktionstypen sind jedoch auch weitere Reaktionen denkbar, die in Gegenwart eines sauren Katalysators durchgeführt werden. Besonders bevorzugt ist der chemische Prozess eine Alkylierung von Salicylsäure oder von substituierten oder unsubstituierten Aromaten. Ganz besonders eignet sich das erfindungsgemäße Verfahren für die Wiederaufarbeitung von Methansulfonsäure, die als Katalysator bei der Herstellung von linearem Alkylbenzol aus Benzol oder substituiertem Benzol mit einem Olefin eingesetzt wird.

Die Alkansulfonsäure, die in dem chemischen Prozess eingesetzt und gemäß dem erfindungsgemäßen Verfahren wiederaufbereitet wird, ist insbesondere Methansulfonsäure.

Ausführungsbeispiele der Erfindung sind in den Figuren dargestellt und werden in der nachfolgenden Beschreibung näher erläutert.

Es zeigen:
- Figur 1: ein Verfahrensfließbild einer Wiederaufarbeitung von Alkansulfonsäure durch Kristallisation,
- Figur 2: ein Verfahrensfließbild einer Wiederaufarbeitung von Alkansulfonsäure durch Kristallisation mit Leichtsiederabtrennung,
- Figur 3: ein Verfahrensfließbild einer Wiederaufarbeitung von Methansulfonsäure durch Destillation und Kristallisation.

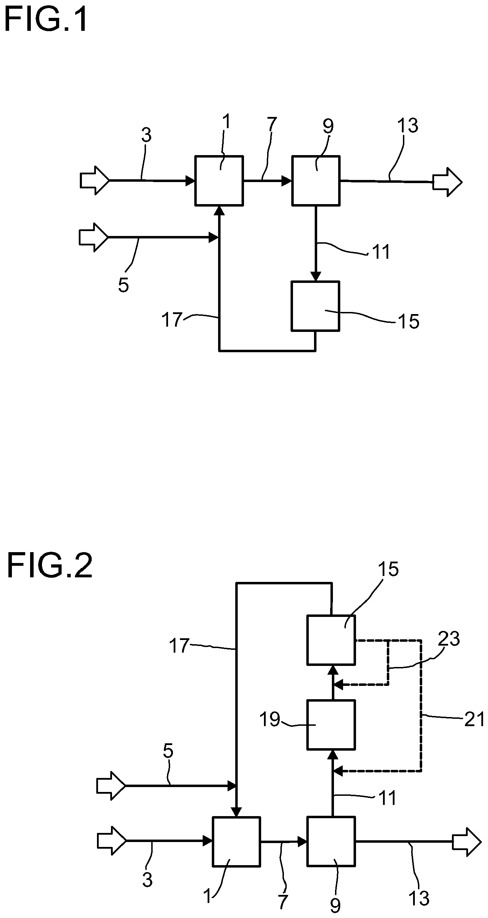

Figur 1 zeigt ein Verfahrensfließbild für einen chemischen Prozess mit einer Wiederaufarbeitung von Alkansulfonsäure durch Kristallisation.

Einem chemischen Prozess 1, zum Beispiel einer Alkylierung zur Herstellung linearer Alkylbenzole, werden Edukte 3 zugeführt. Bei der Herstellung linearer Alkylbenzole werden als Edukte 3 üblicherweise unsubstituiertes oder substituiertes Benzol und lineare Olefine eingesetzt. Die Edukte 3 können dabei zusätzliche Beimengungen, beispielsweise Lösungsmittel, enthalten. Da die Reaktion in Gegenwart eines Katalysators abläuft, muss zusätzlich zu den Edukten 3 ein Katalysator zugegeben werden. Erfindungsgemäß ist der Katalysator Alkansulfonsäure, insbesondere Methansulfonsäure.

Dem chemischen Prozess 1 wird ein Alkansulfonsäure enthaltendes Reaktionsgemisch 7 entnommen. Das Reaktionsgemisch 7 wird einem Separator 9 zugeführt. Im Separator 9 wird das Reaktionsgemisch in einen Alkansulfonsäure enthaltenden Strom 11 und einen Rohproduktstrom 13 getrennt. Der Rohproduktstrom 13 enthält üblicherweise neben dem im chemischen Prozess hergestellten Produkt, beispielsweise des linearen Alkylbenzols noch Edukte, Lösungsmittel und gegebenenfalls bei der Reaktion entstandene Nebenprodukte. Zudem kann auch noch ein kleiner Anteil an Alkansulfonsäure enthalten sein.

Der Alkansulfonsäure enthaltende Strom 11 wird erfindungsgemäß einem Kristallisator 15 zugeführt und durch Kristallisation gereinigt, da in dem Alkansulfonsäure enthaltenden Strom 11 noch Verunreinigungen enthalten sind, die sich bei direkter Rückführung in den chemischen Prozess 1 nachteilig auswirken können, insbesondere durch einen Aktivitätsverlust der als Katalysator eingesetzten Alkansulfonsäure.

Im Kristallisator wird der Alkansulfonsäure enthaltende Strom 11 als Ausgangsschmelze zugeführt und abgekühlt, so dass die Alkansulfonsäure kristallisiert. Das Kristallisat wird entnommen, gegebenenfalls gewaschen, geschmolzen und als Rücklauf 17 wieder in den chemischen Prozess zurückgeführt.

Verluste an Alkansulfonsäure, die zum Beispiel durch Entfernung mit dem Rohproduktstrom 13 oder durch Entfernung von Verunreinigungen aus der Kristallisation auftreten, können über einen Katalysatorzulauf 5 ausgeglichen werden. Hierbei kann die Alkansulfonsäure über den Katalysatorzulauf 5 entweder wie hier dargestellt in den Rücklauf 17 eingeleitet werden oder auch direkt in den chemischen Prozess 1.

Figur 2 zeigt ein Verfahrensfließbild einer Wiederaufarbeitung von Alkansulfonsäure durch Kristallisation mit Leichtsiederabtrennung.

Im Unterschied zu der in Figur 1 dargestellten Ausführungsform umfasst die in Figur 2 dargestellte Ausführungsform zusätzlich eine Leichtsiederabtrennung 19. In diesem Fall wird der dem Separator 9 entnommene, Alkansulfonsäure enthaltende Stoffstrom 11 zunächst in die Leichtsiederabtrennung 19 eingeleitet. In der Leichtsiederabtrennung 19 werden aus dem Alkansulfonsäure enthaltenden Stoffstrom als Verunreinigung enthaltene Leichtsieder vorzugsweise destillativ abgetrennt. Nach der Abtrennung der Leichtsieder wird der Alkansulfonsäure enthaltende Stoffstrom dann als Ausgangsschmelze in den Kristallisator 15 eingeleitet, in dem die Alkansulfonsäure kristallisiert.

Im Kristallisator 15 nicht kristallisierte Alkansulfonsäure enthaltende Mutterlauge kann über eine erste Rückführung 21 ganz oder teilweise in die Leichtsiederabtrennung 19 zurückgeführt werden. Alternativ oder zusätzlich ist es auch möglich, die Mutterlauge ganz oder teilweise über eine zweite Rückführung 23 in den Kristallisator 15 zurückzuführen. Bevorzugt ist jedoch eine Rückführung in die Leichtsiederabtrennung 19, da sich in der Mutterlauge auch zuvor nicht abgetrennte Leichtsieder aufkonzentrieren können.

Ergänzend zur Leichtsiederabtrennung 19 ist auch eine Schwersiederabtrennung vorteilhaft. Diese ist in den Figuren 1 und 2 nicht dargestellt und erfolgt vorzugsweise im Anschluss an die Kristallisation. Zur Schwersiederabtrennung ist es zum Beispiel möglich, die nicht kristallisierte Mutterlauge aus dem Kristallisator 15 zu destillieren, wobei die in der Mutterlauge enthaltene Alkansulfonsäure verdampft wird. Die Schwersieder bleiben dabei im Sumpf in der Flüssigphase zurück. Die verdampfte Alkansulfonsäure kann kondensiert und in den chemischen Prozess 1 zurückgeführt werden. Alternativ ist auch eine Rückführung in die Leichtsiederabtrennung 19 möglich. Dies hat den Vorteil, dass in der Mutterlauge enthaltene Leichtsieder, die bei der destillativen Schwersiederabtrennung ebenfalls in die Gasphase übergehen, noch abgetrennt werden können. Weiterhin ist es auch möglich, die Alkansulfonsäure nach Kondensation entsprechend der zweiten Rückführung 23 in den Kristallisator 15 zurückzuführen. Bevorzugt ist jedoch die Einleitung der kondensierten Alkansulfonsäure aus der Schwersiederabtrennung in den chemischen Prozess 1 oder die Leichtsiederabtrennung 19.

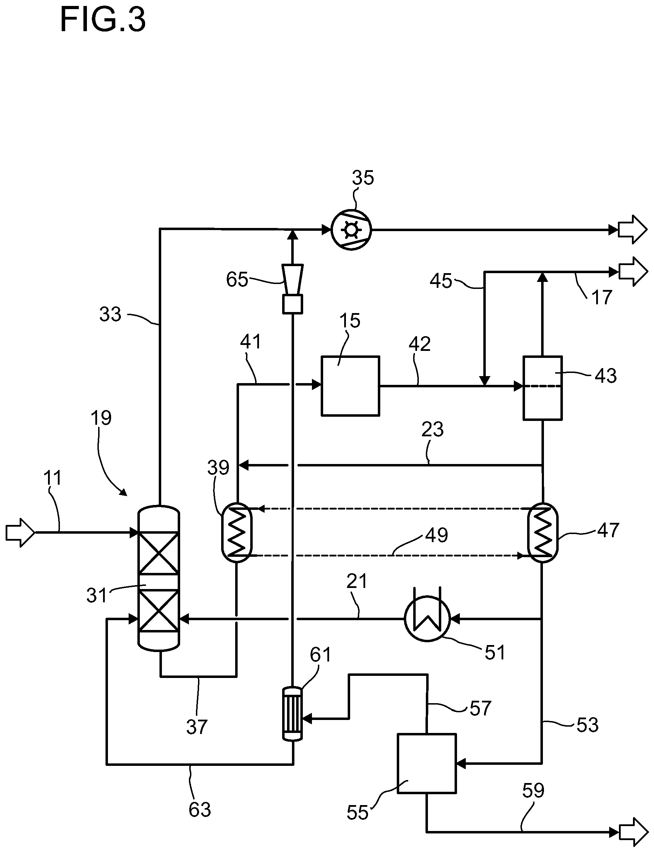

In Figur 3 ist ein Verfahrensfließbild einer Wiederaufarbeitung einer Alkansulfonsäure am Beispiel der Methansulfonsäure durch Destillation und Kristallisation dargestellt.

Ein aus einem chemischen Prozess abgetrennter Alkansulfonsäure enthaltender Strom 11 wird einer Leichtsiederabtrennung 19 zugeführt. Die Leichtsiederabtrennung 19 erfolgt zum Beispiel wie hier dargestellt destillativ. Hierzu wird der Alkansulfonsäure enthaltende Strom 11 im oberen Bereich einer Destillationskolonne 31 aufgegeben. Die Destillationskolonne 31 zur Leichtsiederabtrennung kann geeignete Einbauten enthalten. Als Einbauten können zum Beispiel Böden oder Packungen, wie sie dem Fachmann bekannt sind, vorgesehen sein. Geeignete Böden sind beispielsweise Siebböden, Glockenböden, Tunnelböden oder Lochböden. Packungen, die eingesetzt werden, können geordnete oder ungeordnete Packungen sein. Ungeordnete Packungen enthalten im Allgemeinen Füllkörper, beispielsweise Raschig®-Ringe, Pall-Ringe, Sättel, Berl-Sättel, zylindrische Füllkörper oder beliebige anders geformte Füllkörper. In der Leichtsiederabtrennung 19 wird der Alkansulfonsäure enthaltende Stoffstrom auf eine für die Kristallisation geeignete Konzentration gebracht.

Am Kopf der Destillationskolonne 31 werden Leichtsieder, insbesondere Wasser, als Kopfstrom 33 abgezogen. Der Kopfstrom 33 wird über eine Pumpe 35, beispielsweise eine Flüssigkeitsringpumpe, geführt, mit der der für die Destillation gewünschte Druck erzeugt wird. Anschließend wird der Kopfstrom 33 aus dem Prozess abgezogen und separat aufgearbeitet oder verworfen.

Am Sumpf der Destillationskolonne 31 wird ein Alkansulfonsäure enthaltender Sumpfstrom 37 abgezogen. Die Konzentration an Wasser im Sumpfstrom 37 wird durch die Destillation so eingestellt, dass der Sumpfstrom 37 eine kristallisierbare Konzentration an Alkansulfonsäure enthält.

Da die Kristallisation bei einer Temperatur durchgeführt wird, die wesentlich niedriger ist als die Sumpftemperatur der Destillation, wird der Sumpfstrom 37 in einem ersten Wärmeübertrager 39 abgekühlt. Nach Abkühlung im ersten Wärmeübertrager wird der Sumpfstrom als Ausgangsschmelze 41 dem Kristallisator 15 zugeführt. Die Kristallisation kann dabei als Schichtkristallisation oder auch als Suspensionskristallisation durchgeführt werden. In dem hier beispielhaft dargestellten Prozess ist die Kristallisation eine Suspensionskristallisation.

Dem Kristallisator 15 wird eine Suspension 42 aus Kristallen aus der Alkansulfonsäure und Mutterlauge entnommen und einer Fest-Flüssig-Trennung und einem Wäscher 43 zugeführt, in dem die Alkansulfonsäurekristalle abgetrennt und zur Entfernung der daran anhaftenden Mutterlauge und Verunreinigungen gewaschen werden. Hierzu ist es zum Beispiel möglich, die in Mutterlauge suspendierten Alkansulfonsäurekristalle im Gegenstrom zu einer Waschflüssigkeit, beispielsweise geschmolzenem Kristallisat, zu führen. Die Waschflüssigkeit wäscht die Mutterlauge und Verunreinigungen von den Kristallen ab. Nach Trennung der Kristalle von der Waschflüssigkeit durch ein Fest/Flüssig-Trennverfahren wird gereinigte Alkansulfonsäure gewonnen, die aus dem Prozess entnommen und über den Rücklauf 17 in den chemischen Prozess zurückgeleitet wird.

Ein Teil der gereinigten Alkansulfonsäure kann geschmolzen werden und über eine Leitung 45 als Waschflüssigkeit zurückgeleitet werden.

Die Mutterlauge und Verunreinigungen enthaltende Waschflüssigkeit wird ebenfalls dem Apparat zur Fest-Flüssig-Trennung und Wäscher 43 als an Alkansulfonsäure abgereicherter Strom entnommen. Da dieser Stoffstrom noch einen großen Anteil an Alkansulfonsäure enthält, wird dieser vorzugsweise nicht aus dem Prozess entfernt.

So ist es zum Beispiel möglich, den Waschflüssigkeit und an Alkansulfonsäure abgereicherten, dem Wäscher 43 entnommenen Stoffstrom über die zweite Rückführung 23 in die Schmelzekristallisation 9 zurückzuführen. Hierbei kann entweder der gesamte Strom oder nur ein Teilstrom zurückgeführt werden.

Um bei dieser Rückführung einer unzulässig hohen Aufpegelung von Leichtsiedern entgegen zu wirken, ist es möglich, die Waschflüssigkeit und den an Alkansulfonsäure abgereicherten, dem Wäscher entnommenen Strom über die erste Rückführung 21 in die Destillationskolonne 31 zur Leichtsiederabtrennung 19 zurückzuführen. Dies ist insbesondere dann sinnvoll, wenn im Kristallisator 15 reine Alkansulfonsäure und nicht das Monohydrat der Alkansulfonsäure gewonnen wird. Bei der Kristallisation des Monohydrates lässt sich dagegen eine geeignete Leichtsiederkonzentration alternativ durch Einstellung der Sumpftemperatur der zur Leichtsiederabtrennung 19 eingesetzten Destillationskolonne 31 einstellen. Selbstverständlich ist es auch möglich, einen Teilstrom des Waschflüssigkeit und an Alkansulfonsäure abgereicherte Mutterlauge enthaltenden Stoffstroms über die erste Rückführung 21 in die Leichtsiederabtrennung 19 zurückzuführen und einen weiteren Teilstrom über die zweite Rückführung 23 in den Kristallisator 15.

In der in Figur 3 dargestellten Ausführungsform wird der dem Wäscher 43 entnommene an Alkansulfonsäure abgereicherte Stoffstrom in einem zweiten Wärmeübertrager 47 eingeleitet. Durch eine geeignete Kopplung des ersten Wärmeübertragers 39 und des zweiten Wärmeübertragers 47 wird die niedrige Temperatur des dem Wäscher 43 entnommenen an Alkansulfonsäure abgereicherten Stoffstroms genutzt, um den der Leichtsiederabtrennung entnommenen Alkansulfonsäure enthaltenden Sumpfstrom 37 zu kühlen. Aufgrund der starken Korrosivität der Alkansulfonsäure ist dabei eine direkte Wärmeübertragung vom heißen auf den kalten Strom, wenn überhaupt, nur mit hohem technischem Aufwand möglich. Der Sumpfstrom 37 gibt daher im ersten Wärmeübertrager 39 Wärme an ein Wärmeträgermedium ab. Das Wärmeträgermedium strömt über einen Wärmeträgerkreis 49 in den zweiten Wärmeübertrager 47, in dem dieses die zuvor aufgenommene Wärme an den dem Wäscher 43 entnommenen an Alkansulfonsäure abgereicherten Stoffstrom abgibt und diesen hierdurch erwärmt. Das abgekühlte Wärmeträgermedium fließt dann über den Wärmeträgerkreis 49 wieder zurück in den ersten Wärmeübertrager 39.

Da die Destillation zur Leichtsiederabtrennung bei einer sehr viel höheren Temperatur durchgeführt wird als die Kristallisation ist weiterhin in der ersten Rückführung 21 ein dritter Wärmeübertrager 51 vorgesehen, in dem der zurückgeführte an Alkansulfonsäure abgereicherte Stoffstrom auf die für die Rückführung in die Leichtsiederabtrennung 19 erforderliche Temperatur erwärmt wird.

Um Schwersieder aus dem an Alkansulfonsäure abgereicherten, dem Wäscher 43 entnommenen Stoffstrom zu entfernen, wird zumindest ein Teil der Mutterlauge, der so genannte Schwersiederpurge 53, einem Verdampfer 55 zugeführt. Alternativ ist es auch möglich, den Schwersiederpurge 53 direkt zu verwerfen. Das Verwerfen ist jedoch nur dann sinnvoll, wenn ein großer Teil der Mutterlauge über die erste Rückführung 21 und/oder die zweite Rückführung 23 zurück in den Reinigungsprozess geleitet wurde. Im Verdampfer 55 werden Alkansulfonsäure und Leichtsieder verdampft und als Brüden 57 abgezogen. Die nicht verdampften Anteile werden als aufgearbeiteter Schwersiederpurgestrom 59 aus dem Verdampfer als Flüssigkeit entnommen und einer Weiternutzung oder Entsorgung zugeführt.

Die dem Verdampfer 55 entnommenen Brüden 57 werden entweder wie hier dargestellt in die Leichtsiederabtrennung 19 zurückgeführt oder, vorzugsweise nach Kondensation, in den chemischen Prozess. In der hier dargestellten Ausführungsform strömen die Brüden 57 in einen Teilkondensator 61. Im Teilkondensator 61 kondensiert die in den Brüden enthaltene Alkansulfonsäure und wird als Kondensat 63 in die Leichtsiederabtrennung 19 zurückgeführt. Die Brüden können ohne Teilkondensation nur aufwändig direkt gasförmig in die Leichtsiederabtrennung 19 geführt werden, da der Verdampfer 55 und die zur Leichtsiederabtrennung 19 eingesetzte Destillationskolonne 31 üblicherweise auf unterschiedlichen Druckniveaus arbeiten. Eine alternative Rückführungsmöglichkeit der kondensierten Brüden besteht nach Abkühlung in der Nutzung als Waschflüssigkeit für den Wäscher 43.

Der nicht kondensierte und Leichtsieder enthaltende Anteil wird dem Teilkondensator 61 gasförmig entnommen und zusammen mit dem Leichtsieder enthaltenden Stoffstrom 33 aus der Destillationskolonne 31 aus dem Prozess abgezogen.

Um den Druck des Kondensats 63, das in die Destillationskolonne 31 zurückgeleitet wird, zu reduzieren, ist es möglich, in dem gasförmig dem Teilkondensator 61 entnommenem Stoffstrom eine Strahlpumpe 65 vorzusehen. Alternativ zur Strahlpumpe kann auch jede andere dem Fachmann bekannte Vorrichtung eingesetzt werden, mit der ein Gas gefördert und ein Unterdruck erzeugt werden kann. Durch den Einsatz der Strahlpumpe 65 wird der Druck im Teilkondensator 61 verringert, so dass auch das Kondensat 63 den Teilkondensator 61 mit einem niedrigeren Druck als dem Druck des Verdampfers 55 verlässt.

Aus Gründen der Energieeinsparung ist es sinnvoll, den Schwersiederpurge 53 so klein wie möglich zu wählen. Die Verringerung der Menge des Schwersiederpurges 53 wird dadurch nach unten begrenzt, dass noch ausreichende Mengen an Schwersieder aus dem Kreisprozess ausgeschleust werden, um deren Konzentration unterhalb der Konzentration zu halten, ab der sie die Kristallisation stören.

### Beispiele

### Beispiel 1

Zur Herstellung von Dodecylbenzol mit 100%iger Methansulfonsäure als Katalysator wurden 164 g Benzol, 50 g 1-Dodecen und 283 g n-Octan zusammen mit 769 g Methansulfonsäure (Lutropur® 100 der BASF SE) in einen doppelwandigen Glasreaktor mit einem Fassungsvolumen von 2,5 l eingefüllt. Der Glasreaktor war ausgerüstet mit einem Rührer, einem Kondensator und einem Thermoelement. Der Versuch wurde unter Argon als Inertgas durchgeführt. Nach einem Vermischen der Lösung für 2 Minuten bei 1000 min⁻¹ wurde der Rührer angehalten. Innerhalb von etwa 120 Sekunden erfolgte eine Phasentrennung. Nach Auftrennung der Phasen wurde eine Probe der organischen Phase als Startprobe entnommen. Anschließend wurde die Reaktionsmischung unter Rühren mit 1000 min⁻¹ innerhalb von 30 Minuten auf 60 °C erhitzt und die Mischung dann weitere fünf Stunden bei dieser Temperatur gerührt. Nach jeder Stunde wurde der Rührer kurz angehalten und nach Phasentrennung eine Probe der organischen Phase entnommen. Nach beendeter Reaktion wurde der Rührer abgestellt und die Reaktionsmischung auf Raumtemperatur abgekühlt. Dabei trennten sich die Phasen auf. Die obere hydrophobe Phase war klar und farblos. Die untere, methansulfonsäurehaltige Phase war leicht braun verfärbt. Sie wurde abgelassen. Man erhält 428 g hydrophobe Phase und 777 g hydrophile, methansulfonsäurehaltige Phase. Die Proben der organischen Phase wurden mittels Gaschromatographie analysiert und so der Reaktionsfortschritt betrachtet. Die Abnahme der Dodecenkonzentration und die Bildung von Dodecylbenzolisomeren wurden beobachtet. Das Fläche der GC-Signale der Dodecenisomere, der Dodecylbenzolisomere und von n-Octan wurde vermessen und das Verhältnis von Dodecenisomeren/Octan bzw. Dodecylbenzolisomeren/-Octan gebildet. Zur Beobachtung der Abnahme der Dodecenkonzentration wurde auf den Wert der Startprobe normiert.

### Beispiele 2 bis 8

Die Beispiele beschreiben die Herstellung von Dodecylbenzol mit Rückführung der hydrophilen, methansulfonsäurehaltigen Phase.

Die Versuche wurden analog Beispiel 1 durchgeführt. Anstelle der 769 g frischer Methansulfonsäure wurde jedoch die hydrophile, methansulfonsäurehaltige Phase aus dem vorhergehenden Experiment eingesetzt. Verluste an Methansulfonsäure, die durch Probennahme und Analytik der Phase entstanden sind, wurden mit 100%iger frischer Methansulfonsäure ersetzt. So lagen nach dem in Beispiel 1 beschriebenen Versuch und der Analytik der methansulfonsäurehaltigen hydrophilen Phase noch 748 g der hydrophilen Phase vor. Zu dieser Menge wurde 21 g 100%iger Methansulfonäure zugegeben um den Verlust auszugleichen.

Aussehen, Methansulfonsäuregehalt und Wassergehalt der hydrophilen Phase nach den Versuchen sind in Tabelle 1 wiedergegeben.

Nach sieben Rückführzyklen war die hydrophile Phase gelb/braun gefärbt und die hydrophobe Produktphase wies eine rötliche Färbung auf. Die Versuche zeigen, dass sich während der Methansulfonsäure-katalysierten Reaktion von Dodecen mit Benzol gefärbte Verbindungen bilden, die sich in der Methansulfonsäure-haltigen hydrophilen Phase anreichern. Ohne Entfernung dieser Komponenten kommt es bei Rückführung der Methansulfonsäure ebenfalls zu Verfärbungen der hydrophoben Dodecylbenzol enthaltenden Phase.

Es hat sich gezeigt, dass sich der Wassergehalt in der Reaktionsmischung bei jeder Rückführung leicht erhöht hat. Bei der Untersuchung der jeweils stündlich entnommenen organischen Phase hat sich gezeigt, dass ein Wassergehalt in der methansulfonsäurehaltigen Phase von mehr als 0,25 Gew.-% zu einem signifikanten Abfall der katalytischen Aktivität führt.

### Beispiele 9 bis 12

Die Beispiele 9 bis 12 beschreiben die Herstellung von Dodecylbenzol mit unterschiedlichen Wasserkonzentrationen in der methansulfonsäurehaltigen Phase.

Die Versuche wurden analog Beispiel 1 durchgeführt. Zur Methansulfonsäure wurde jedoch vor jedem Versuch eine kleine Menge Wasser zugegeben. Nach Phasentrennung der Reaktionsmischung wurde der Wassergehalt in der hydrophilen, methansulfonsäurehaltigen Phase analysiert. Die Wassergehalte der einzelnen Versuche sind ebenfalls in Tabelle 1 wiedergegeben.

Die Umsatzkurven von Dodecen in den Versuchen und die Bildung von Dodecylbenzol zeigen deutlich den Einfluss von Wasser auf die katalytische Aktivität der Methansulfonsäure. Mit zunehmendem Wassergehalt ist auch hier der Umsatz merklich zurückgegangen.

**Tabelle 1: Zusammensetzung der hydrophilen Phase**

| Beispiel | Methansulfonsäure Rückführung aus Beispiel | Methansulfonsäuregehalt der hydrophilen Phase (Gew.-%) | Wassergehalt der hydrophilen Phase (Gew.-%) | Aussehen |
|---|---|---|---|---|
| 1 | Start | 97,6 | 0,10 | leicht gelblich |
| 2 | 1 | 97,4 | 0,12 | gelblich |
| 3 | 2 | 97,3 | 0,15 | Farbe wird intensiver gelb |
| 4 | 3 | 97,4 | 0,17 | |
| 5 | 4 | 97,2 | 0,25 | |
| 6 | 5 | 97,2 | 0,27 | |
| 7 | 6 | 97,0 | 0,29 | |
| 8 | 7 | 97,1 | 0,43 | gelb/braun |
| 9 | - | n. b. | 0,30 | leicht gelblich |
| 10 | - | n. b. | 0,39 | leicht gelblich |
| 11 | - | n. b. | 0,60 | leicht gelblich |
| 12 | - | n. b. | 1,10 | leicht gelblich |

| | | | | |
|---|---|---|---|---|
| n. b.: nicht bestimmt. | | | | |

### Beispiel 13

Das Beispiel beschreibt die Herstellung von Dodecylbenzol und die destillative Entfernung des Wassers aus der hydrophilen Phase.

164 g Benzol, 50 g 1-Dodecen und 94 g n-Octan wurden zusammen mit 765,2 g Methansulfonsäure (Lutropur® 100 der BASF SE) und 3,8 g Wasser (entsprechend 0,5 Gew.-% Wasser in der hydrophilen Phase) in einen doppelwandigen Glasreaktor mit einem Fassungsvolumen von 2,5 l eingefüllt. Der Glasreaktor war ausgerüstet mit einem Rührer, einem Kondensator und einem Thermoelement. Der Versuch wurde unter Argon als Inertgas durchgeführt. Nach einem Vermischen der Lösung für 2 Minuten bei 1000 min⁻¹ wurde der Rührer angehalten. Nach Auftrennung der Phasen wurde eine Probe der organischen Phase als Startprobe entnommen. Anschließend wurde die Reaktionsmischung unter Rühren mit 1000 min⁻¹ innerhalb von 45 Minuten auf 80 °C erhitzt und die Mischung dann weitere fünf Stunden bei dieser Temperatur gerührt. Nach jeder Stunde wurde der Rührer kurz angehalten und nach Phasentrennung eine Probe der organischen Phase entnommen. Nach beendeter Reaktion wurde der Rührer abgestellt und die Reaktionsmischung auf Raumtemperatur abgekühlt. Dabei trennten sich die Phasen auf. Die untere, methansulfonsäurehaltige Phase war leicht gelb verfärbt. Sie wurde abgelassen. Man erhält 233 g hydrophobe Phase und 772 g hydrophile, methansulfonsäurehaltige Phase. Die Phasen wurden wie unter Beispiel 1 beschrieben analysiert.

757 g der leicht gelben hydrophilen Phase mit einem Wassergehalt von 0,7 Gew.-%, wurden in einem Rotationsverdampfer bei 160 °C bei einem Druck von 4 mbar (abs) andestilliert. Dabei wurden 34 g Destillat im Vorlagekolben aufgefangen. Während der Destillation kommt es zu einer Dunkelfärbung im Sumpf. Im Sumpf verblieben nach beendeter Destillation 689 g gelb gefärbte hydrophile Phase. Der Wassergehalt der verbliebenen hydrophilen Phase betrug 0,05 Gew.-%.

### Beispiele 14 bis 21

Die Beispiele 14 bis 21 beschreiben die Herstellung von Dodecylbenzol mit rückgeführter hydrophiler Phase nach destillativer Entfernung des Wassers in einer Laborapparatur.

Die Versuche wurden analog der Beschreibung von Beispiel 13 durchgeführt. Anstatt den 765,2 g frischer Methansulfonsäure wurde jedoch die hydrophile, methansulfonsäurehaltige Phase aus dem vorhergehenden Experiment eingesetzt. Verluste an Methansulfonsäure, die durch Probennahme und Analytik der Phase entstanden sind, wurden durch 100%ige Methansulfonsäure ersetzt.

Nach Durchführung des Beispiel 19 und chemischer Analyse der methansulfonsäurehaltigen hydrophilen Phase betrug die Menge der hydrophilen Phase noch 675 g. Zu dieser Menge wurden 90,2 g 100%ige Methansulfonsäure zum Verlustausgleich zugegeben.

Die Destillationsparameter aus Beispiel 19 sind hier exemplarisch beschrieben. In diesem Versuch wurden 780,6 g hydrophile Phase eingesetzt. Die Sumpftemperatur am Ende der Destillation betrug 143 °C. Die Kopftemperatur betrug 135 °C bei einem Kopfdruck von 2 mbar. Es wurden 25,3 g einer ersten Kopffraktion erhalten, die hauptsächlich Benzol enthielt und 58,0 g einer zweiten Kopffraktion, die im Wesentlichen Wasser und Methansulfonsäure enthielt. Während der Destillation verfärbte sich der Sumpf stark dunkel. Die Wasserkonzentration im Sumpf betrug 0,4 Gew.-% (nach Karl-Fischer Titration). Der Sumpf (677,3 g) wurde in Versuch 21 eingesetzt. Die Verluste an Methansulfonsäure wurden mit 100%iger Methansulfonsäure ausgeglichen.

Nach Versuch 21 und Phasentrennung wurden 236,8 g einer leicht bräunlich verfärbten, trüben hydrophoben Phase und 775,6g einer leicht trüben, dunkelbraun verfärbten hydrophilen Methansulfonsäure-haltigen Phase erhalten.

Nach mehrfacher Destillation vertiefte sich die Farbe der hydrophilen methansulfonsäurehaltigen Phase stark. Zusätzlich entstanden fein verteilte Feststoffpartikel. Farbige Komponenten führten auch in der hydrophoben Phase zu einer merklichen Dunkelfärbung.

Die Versuche zeigen, dass unter den Bedingungen einer Destillation Methansulfonsäure mit Reaktionsprodukten aus dem LAB-Prozess chemisch reagiert. Die Reaktionsprodukte aus der Reaktion von Methansulfonsäure sind stark dunkel gefärbt und können bei wiederholter Destillation, insbesondere bei Temperaturen oberhalb von 150 °C, unlösliche Partikel oder Öle bilden.

Obwohl die Destillationsbedingungen zur Abtrennung des Wassers unter 145°C gewählt waren, war die Reaktion der Methansulfonsäure mit aromatischen Olefinen nicht zu vermeiden. Durch Absenkung des Destillationsdruckes kann zwar prinzipiell bei niedrigeren Temperaturen mit verringerter Bildung von Verunreinigungen destilliert werden, allerdings erfordert dieses Verfahren sehr niedrige Drucke, beispielsweise unter 10 mbar, und einen entsprechend höheren Aufwand.

Die entstehenden Reaktionsprodukte lassen eine Destillation, die zu sehr niedrigen Wasserkonzentrationen von weniger als1 Gew.-% in der rückgeführten Methansulfonsäure führt, in einem rückgeführten LAB-Prozess als einen nicht geeigneten Prozessschritt erscheinen. Demgemäß ist eine Destillation kein geeignetes Mittel zum Recycling von Methansulfonsäure.

### Beispiel 22

Das Beispiel beschreibt die Herstellung von Dodecylbenzol mit rückgeführter hydrophiler Phase aus Versuch 21.

Der Versuch wurde analog Beispiel 1 durchgeführt. Anstatt der 769 g frischer Methansulfonsäure wurde jedoch die hydrophile, methansulfonsäurehaltige Phase (681 g) aus Beispiel 22 eingesetzt. Zu dieser Menge wurden 88 g 100%iger Methansulfonsäure zugegeben um den Verlust auszugleichen. Im Gegensatz zu Beispiel 1 erfolgten die Phasentrennungen in diesen Versuchen jedoch nicht innerhalb von 120 Sekunden, sondern benötigten mindestens 10 Minuten. Nach beendeter Reaktion bildete sich zwischen der tief dunkel gefärbten hydrophilen und der bräunlichen hydrophoben Phase eine intermediäre Phase aus. Alle drei Phasen wurden isoliert. Die intermediäre Phase trennte sich erst nach längerem Stehen für mehrere Stunden in eine hydrophobe und hydrophile Phase auf.

Das Beispiel zeigt, dass sich oberflächenaktive und farbgebende Verbindungen in der hydrophilen Phase bilden und anreichern können.

### Beispiel 23

Das Beispiel beschreibt die Herstellung von Dodecylbenzol mit 100%iger Methansulfonsäure als Katalysator.

78,7 g Benzol, 24,0 g 1-Dodecen und 135,8 g n-Octan wurden zusammen mit 370 g durch Kristallisation gereinigter Methansulfonsäure in einen doppelwandigen Glasreaktor mit einem Fassungsvolumen von 2,5 l eingefüllt. Der Glasreaktor war ausgerüstet mit einem Rührer, einem Kondensator und einem Thermoelement. Der Versuch wurde unter Argon als Inertgas durchgeführt. Nach kurzem Vermischen wurde der Rührer angehalten. Es erfolgte innerhalb von etwa 30 Sekunden eine Phasentrennung. Nach Auftrennung der Phasen wurde eine Probe der organischen Phase entnommen. Anschließend wurde die Reaktionsmischung unter Rühren auf 60°C erhitzt und die Mischung weitere fünf Stunden gerührt. Nach jeder Stunde wurde der Rührer kurz angehalten und nach Phasentrennung eine Probe der organischen Phase entnommen. Nach beendeter Reaktion wurde der Rührer abgestellt und die Reaktionsmischung auf Raumtemperatur abgekühlt, Dabei trennten sich die Phasen auf. Die obere hydrophobe Phase ist klar und farblos. Die untere, methansulfonsäurehaltige Phase ist leicht braun verfärbt und trüb. Sie wurde abgelassen und auf ihren Wasserwert analysiert. Sie enthielt 0,22 Gew.-% Wasser.

### Beispiel 24

Das Beispiel beschreibt die Herstellung von Dodecylbenzol mit Methansulfonsäure, die durch Kristallisation gereinigt wurde.

78,7 g Benzol, 24,0 g 1-Dodecen und 135,8 g n-Octan wurden zusammen mit 370 g durch Kristallisation gereinigte Methansulfonsäure (aus Beispiel 25) in einen doppelwandigen Glasreaktor mit einem Fassungsvolumen von 2,5 l eingefüllt. Der Glasreaktor war ausgerüstet mit einem Rührer, einem Kondensator und einem Thermoelement. Der Versuch wurde unter Argon als Inertgas durchgeführt. Nach kurzem Vermischen wurde der Rührer angehalten. Es erfolgte innerhalb von etwa 30 Sekunden eine Phasentrennung. Nach Auftrennung der Phasen wurde eine Probe der organischen Phase entnommen. Anschließend wurde die Reaktionsmischung unter Rühren auf 60 °C erhitzt und die Mischung weitere fünf Stunden gerührt. Nach jeder Stunde wurde der Rührer kurz angehalten und nach Phasentrennung eine Probe der organischen Phase entnommen. Nach beendeter Reaktion wurde der Rührer abgestellt und die Reaktionsmischung auf Raumtemperatur abgekühlt. Dabei trennten sich die Phasen auf. Die obere hydrophobe Phase ist klar und farblos. Die untere, methansulfonsäurehaltige Phase ist leicht braun verfärbt und trüb. Sie wurde abgelassen und auf ihren Wasserwert analysiert. Sie enthielt 0,15 Gew.-% Wasser. Der Vergleich des Reaktionsverlaufes von Beispiel 23 und Beispiel 24 zeigt keine Abweichung.

Beispiel 24 zeigt, dass sich Methansulfonsäure durch Kristallisation soweit aufreinigen lässt, dass es eine Qualität erreicht, die zu einer Rückführung in einen mit Methansulfonsäure katalysierten Prozess geeignet ist.

### Beispiel 25

Das Beispiel beschreibt die Kristallisation von Methansulfonsäure aus der verunreinigten methansulfonsäurehaltigen Phase aus der Produktion von linearem Alkylbenzol.

Aus 1510 g verunreinigte Methansulfonsäure aus einer im Labor nachgestellten Produktion von linearem Alkylbenzol wurden Leichtsieder und Wasser mittels Destillation bis zu einer Restkonzentration von Wasser von 0,35 Gew.-% bei einem Druck unter gleichen Bedingungen wie in Beispiel 19 entfernt. Der nach dieser Destillation tief dunkel eingefärbte Sumpf der Destillationskolonne wurde danach in einen 1-Liter-Doppelmantelrührbehälter mit einem Durchmesser von 150 mm mit einem wandgängigen Wendelrührer eingefüllt. Danach wurde das Gemisch von 15 °C mit einer Abkühlrate von 1 K/h auf 2,8°C abgekühlt. Die Temperatur wurde in dem Gemisch mit einem eingetauchten PT100 Thermoelement gemessen. Bei der Abkühlung entstanden in dem Gemisch Kristalle, die durch Rühren bei 250 min⁻¹ in Suspension gehalten wurden. Die erhaltenen Kristalle in der Suspension wurden auf einem Druckfilter bei ca. 3,5 bar innerhalb von weniger als 1 Minute abgetrennt. Dabei wurde die Mutterlauge der Kristallisation aufgefangen. Das Kristallisat wurde mit reiner Methansulfonsäure im Verhältnis von 1:1 gewaschen und anschließend geschmolzen. Die Waschlösung wurde ebenfalls separat aufgefangen Das geschmolzene Kristallisat ist eine farblose Flüssigkeit mit einem Wassergehalt von 0,10 Gew.-%.

Die erhaltene Mutterlauge der Kristallisation, das Waschfiltrat und das gewaschene, geschmolzene Kristallisat wurden mit einem PerkinElmer Lambda 900® UV/Vis Spektrometer vermessen. Als Referenz wurde Reinstwasser aus einer Referenzwasseranlage der Fa. Millipore vom Typ Milli-Q® mit einem elektrischen Widerstand über 18,2 MΩ und einem TOC-Gehalt kleiner 5ppb verwendet.

Die Spektralanalyse wurde bei Raumtemperatur mit einer Messgeschwindigkeit von
267 nm/min, einer Integrationszeit von 0,20 s, einem Datenintervall von 1nm, einer Spaltweite von 2nm und einem Wellenlängenbereich von 200-800 nm in einer Quarzglasküvette mit einem Durchmesser von 0,1 cm durchgeführt.

Die Spektralanalyse ergab folgende Extinktion bei 400 nm:

| | |
|---|---|
| Mutterlauge der Kristallisation | E₄₀₀ = 1,9 |
| Waschfiltrat | E₄₀₀ = 0,8 |
| Kristallisat | E₄₀₀ = 0,03 |

Aus den erhaltenen Absorptionsspektren ist deutlich ersichtlich, dass farbgebende Komponenten, die durch nicht schonende Destillationsbedingungen erzeugt werden, nahezu vollständig durch Kristallisation aus Methansulfonsäure entfernt werden können. Ein Vergleich der Wasserwerte von Ausgangsmaterial und Methansulfonsäure-Kristallisat zeigt, dass auch Leichtsieder wie Wasser aus der Methansulfonsäure durch Kristallisation entfernt werden können.

### Beispiel 26

Das Beispiel beschreibt die Reinigung von Methansulfonsäure durch Kristallisation zur Entfernung von Wasser.

Methansulfonsäure-Ausgangsschmelze mit einem Methansulfonsäure-Gehalt von 96 Gew.-% und einem Anteil von 3.0 Gew.-% Wasser sowie weiteren Verunreinigungen und einer Temperatur von 15°C wurde bei Atmosphärendruck in einem Doppelmantelrührbehälter mit einem Fassungsvermögen von 1 l und einem Durchmesser von 150 mm mit einem wandgängigen Wendelrührer eingefüllt. Danach wurde die Ausgangsschmelze mit einer Abkühlrate von 1 K/h auf 6,5°C abgekühlt. Die Temperatur wurde in der Ausgangsschmelze mit einem PT100-Thermoelement gemessen. Bei der Abkühlung entstanden Kristalle, die durch Rühren mit 150 ppm in Suspension gehalten wurden. Die erhaltenen Kristalle in der Suspension wurden auf einem Druckfilter bei ca. 3,5 bar innerhalb von weniger als 1 Minute abgetrennt. Dabei wurde die Mutterlauge der Kristallisation aufgefangen. Das Kristallisat wurde mit reiner Methansulfonsäure im Verhältnis von 1:1 gewaschen und anschließend geschmolzen. Die Waschlösung wurde ebenfalls separat aufgefangen Das geschmolzene Methansulfonsäure-Kristallisat hat einen Wassergehalt von 0,27 Gew.-%.

Das Beispiel zeigt, dass die Kristallisation eine geeignete Methode zur Entfernung von Wasser aus einem Methansulfonsäure- Wasser-Gemisch ist.

### Bezugszeichenliste

- 1: chemischer Prozess
- 3: Edukte
- 5: Katalysatorzulauf
- 7: Reaktionsgemisch
- 9: Separator
- 11: Alkansulfonsäure enthaltender Strom
- 13: Rohproduktstrom
- 15: Kristallisator
- 17: Rücklauf
- 19: Leichtsiederabtrennung
- 21: erste Rückführung
- 23: zweite Rückführung
- 31: Destillationskolonne
- 33: Kopfstrom
- 35: Pumpe
- 37: Sumpfstrom
- 39: erster Wärmeübertrager
- 41: Ausgangsschmelze
- 42: Suspension
- 43: Wäscher
- 45: Leitung
- 47: zweiter Wärmeübertrager
- 49: Wärmeträgerkreis
- 51: dritter Wärmeübertrager
- 53: Schwersiederpurge
- 55: Verdampfer
- 57: Brüden
- 59: Schwersiederpurgestrom
- 61: Teilkondensator
- 63: Kondensat
- 65: Strahlpumpe

## Patentansprüche

1. Verfahren zur Wiederaufarbeitung von Alkansulfonsäure, die in einem chemischen Prozess als Agens, Katalysator oder Lösungsmittel eingesetzt wird, umfassend folgende Schritte:
(a) Abtrennen eines Alkansulfonsäure enthaltenden Stromes aus einem Reaktionsgemisch, das in dem chemischen Prozess anfällt,
(b) Zufuhr des Alkansulfonsäure enthaltenden Stromes als Ausgangsschmelze in eine Schmelzekristallisation, wobei sich Kristalle der Alkansulfonsäure, von Hydraten der Alkansulfonsäure oder einer Mischung aus beiden suspendiert in Mutterlauge bilden,
(c) Durchführen einer Fest-Flüssig-Trennung, um die Kristalle aus der Mutterlauge abzutrennen,
(d) Gegebenenfalls Waschen der Kristalle, um an den Kristallen anhaftende Mutterlauge zu entfernen,
(e) Rückführen der aus der Mutterlauge abgetrennten und gegebenenfalls gewaschenen Kristalle in den chemischen Prozess.

2. Verfahren gemäß Anspruch 1, **dadurch gekennzeichnet, dass** das Reaktionsgemisch in Schritt (a) zweiphasig ist und der Alkansulfonsäure enthaltende Strom durch Phasentrennung aus dem Reaktionsgemisch abgetrennt wird.

3. Verfahren gemäß Anspruch 1 oder 2, **dadurch gekennzeichnet, dass** die Mutterlauge nach Abtrennung der Kristalle in Schritt (c) und/oder die in Schritt (b) anfallende Mutterlauge zumindest teilweise in die Schmelzekristallisation in Schritt (b) zurückgeführt wird.

4. Verfahren gemäß Anspruch 1 oder 2, **dadurch gekennzeichnet, dass** der Alkansulfonsäure enthaltende Strom vor Zufuhr in die Schmelzekristallisation in Schritt (b) zur Abtrennung von Leichtsiedern destilliert wird, wobei die Leichtsieder am Kopf einer Destillationskolonne abgezogen werden und ein Alkansulfonsäure, Schwersieder und Reste an Leichtsiedern enthaltender Stoffstrom am Sumpf der Destillationskolonne entnommen wird und der Alkansulfonsäure, Schwersieder und Reste an Leichtsiedern enthaltende Strom der Schmelzekristallisation in Schritt (b) zugeführt wird.

5. Verfahren gemäß Anspruch 4, **dadurch gekennzeichnet, dass** die Mutterlauge nach Abtrennung der Kristalle in Schritt (c) und/oder die in Schritt (b) anfallende Mutterlauge zumindest teilweise in die Schmelzekristallisation oder in die Destillation zur Abtrennung von Leichtsiedern zurückgeführt wird.

6. Verfahren gemäß Anspruch 1 oder 2, **dadurch gekennzeichnet, dass** die Kristalle aus Schritt (c) oder aus Schritt (d) einer Destillation zur Wasserabtrennung zugeführt werden.

7. Verfahren gemäß Anspruch 1 oder 2, **dadurch gekennzeichnet, dass** die Mutterlauge nach Abtrennung der Kristalle in Schritt (c) und/oder die in Schritt (b) anfallende Mutterlauge zumindest teilweise einer Schwersiederabtrennung zugeführt wird, in der Alkansulfonsäure durch Verdampfung abgetrennt wird.

8. Verfahren gemäß Anspruch 7, **dadurch gekennzeichnet, dass** die durch Verdampfung abgetrennte Alkansulfonsäure in den chemischen Prozess zurückgeführt wird.

9. Verfahren gemäß Anspruch 8, **dadurch gekennzeichnet, dass** die durch Verdampfung abgetrennte Alkansulfonsäure vor der Rückführung in den chemischen Prozess zumindest teilkondensiert wird.

10. Verfahren gemäß Anspruch 7, **dadurch gekennzeichnet, dass** die Mutterlauge nach dem Entfernen der Schwersieder teilkondensiert wird und der kondensierte Anteil in die Destillation zur Abtrennung der Leichtsieder oder in die Schmelzekristallisation in Schritt (b) zurückgeführt wird und der nicht kondensierte Anteil als Leichtsieder aus dem Prozess ausgeschleust wird.

11. Verfahren gemäß einem der Ansprüche 1 bis 10, **dadurch gekennzeichnet, dass** die Alkansulfonsäure Methansulfonsäure ist.

12. Verfahren gemäß einem der Ansprüche 1 bis 11, **dadurch gekennzeichnet, dass** der chemische Prozess eine Alkylierung oder eine Veresterungsreaktion ist.

13. Verfahren gemäß einem der Ansprüche 1 bis 12, **dadurch gekennzeichnet, dass** der chemische Prozess eine Alkylierung von Salicylsäure oder von substituierten oder unsubstituierten Aromaten ist.

14. Verfahren gemäß Anspruch 13, **dadurch gekennzeichnet, dass** der chemische Prozess die Herstellung von linearem Alkylbenzol aus Benzol oder substituiertem Benzol mit einem Olefin in Gegenwart von Alkansulfonsäure als Katalysator ist.

15. Verfahren gemäß einem der Ansprüche 1 bis 14, **dadurch gekennzeichnet, dass** die Schmelzekristallisation eine Suspensionskristallisation oder eine Schichtkristallisation ist.

## Claims

1. A method of reprocessing alkanesulfonic acid employed in a chemical process as an agent, catalyst or solvent and comprising the steps of:
(a) removing an alkanesulfonic acid-comprising stream from a reaction mixture generated in the chemical process,
(b) sending the alkanesulfonic acid-comprising stream into a melt crystallization as the starting melt to form crystals of the alkanesulfonic acid, of hydrates of the alkanesulfonic acid or of a mixture of both suspended in mother liquor,
(c) performing a solid-liquid separation to remove the crystals from the mother liquor,
(d) optionally washing the crystals to remove mother liquor adhering to the crystals.
(e) recycling the washed or unwashed crystals removed from the mother liquor into the chemical process.

2. The method according to claim 1, wherein the reaction mixture in step (a) is biphasic and the alkanesulfonic acid-comprising stream is removed from the reaction mixture by phase separation.

3. The method according to claim 1 or 2, wherein the mother liquor after removal of the crystals in step (c) and/or the mother liquor generated in step (b) is/are at least partly recycled into the melt crystallization in step (b).

4. The method according to claim 1 or 2, wherein the alkanesulfonic acid-comprising stream before being sent into the melt crystallization in step (b) is distilled to remove low boilers, wherein the low boilers are drawn off at the top of a distillation column and a material stream comprising alkanesulfonic acid, high boilers and residual low boilers is withdrawn at the bottom of the distillation column and the stream comprising alkanesulfonic acid, high boilers and residual low boilers is sent to the melt crystallization in step (b).

5. The method according to claim 4, wherein the mother liquor after removal of the crystals in step (c) and/or the mother liquor generated in step (b) is/are at least partly recycled into the melt crystallization or into the distillation for removing low boilers.

6. The method according to claim 1 or 2, wherein the crystals from step (c) or from step (d) are sent to a distillation for water removal.

7. The method according to claim 1 or 2, wherein the mother liquor after removal of the crystals in step (c) and/or the mother liquor generated in step (b) is/are at least partly sent to a high boilers removal in which alkanesulfonic acid is removed by evaporation.

8. The method according to claim 7, wherein the alkanesulfonic acid removed by evaporation is recycled into the chemical process.

9. The method according to claim 8, wherein the alkanesulfonic acid removed by evaporation is at least partially condensed before being recycled into the chemical process.

10. The method according to claim 7, wherein the mother liquor after removal of the high boilers is partially condensed and the condensed portion is recycled into the distillation for removing the low boilers or into the melt crystallization in step (b) and the uncondensed portion is discharged from the process as low boilers.

11. The method according to any of claims 1 to 10, wherein the alkanesulfonic acid is methanesulfonic acid.

12. The method according to any of claims 1 to 11, wherein the chemical process is an alkylation or an esterification reaction.

13. The method according to any of claims 1 to 12, wherein the chemical process is an alkylation of salicylic acid or of substituted or unsubstituted aromatics.

14. The method according to claim 13, wherein the chemical process is the production of linear alkylbenzene from benzene or substituted benzene with an olefin in the presence of alkanesulfonic acid as catalyst.

15. The process according to any of claims 1 to 14, wherein the melt crystallization is a suspension crystallization or a layer crystallization.

## Revendications

1. Procédé de retraitement d'un acide alcane-sulfonique, qui est utilisé en tant qu'agent, catalyseur ou solvant dans un processus chimique, comprenant les étapes suivantes :
(a) la séparation d'un courant contenant un acide alcane-sulfonique d'un mélange réactionnel produit dans le cadre du processus chimique,
(b) l'introduction du courant comprenant l'acide alcane-sulfonique sous la forme d'une masse fondue de départ dans une cristallisation de masse fondue, des cristaux de l'acide alcane-sulfonique, d'hydrates de l'acide alcane-sulfonique ou d'un mélange des deux se formant en suspension dans une liqueur mère,
(c) la réalisation d'une séparation solide-liquide afin de séparer les cristaux de la liqueur mère,
(d) éventuellement le lavage des cristaux afin d'éliminer la liqueur mère qui adhère sur les cristaux,
(e) le recyclage des cristaux séparés de la liqueur mère et éventuellement lavés dans le processus chimique.

2. Procédé selon la revendication 1, **caractérisé en ce que** le mélange réactionnel à l'étape (a) est biphasé, et le courant contenant l'acide alcane-sulfonique est séparé du mélange réactionnel par séparation de phases.

3. Procédé selon la revendication 1 ou 2, **caractérisé en ce que** la liqueur mère après la séparation des cristaux à l'étape (c) et/ou la liqueur mère formée à l'étape (b) sont au moins partiellement recyclées dans la cristallisation de masse fondue à l'étape (b).

4. Procédé selon la revendication 1 ou 2, **caractérisé en ce que** le courant contenant l'acide alcane-sulfonique est distillé avant l'introduction dans la cristallisation de masse fondue de l'étape (b) pour la séparation de composants de point d'ébullition faible, les composants de points d'ébullition faible étant soutirés à la tête d'une colonne de distillation, et un courant de matière contenant l'acide alcane-sulfonique, les composants de point d'ébullition élevé et les résidus des composants de point d'ébullition faible étant soutiré au fond de la colonne de distillation, et le courant contenant l'acide alcane-sulfonique, les composants de point d'ébullition élevé et les résidus des composants de point d'ébullition faible étant introduit dans la cristallisation de masse fondue de l'étape (b).

5. Procédé selon la revendication 4, **caractérisé en ce que** la liqueur mère après la séparation des cristaux à l'étape (c) et/ou la liqueur mère formée à l'étape (b) sont au moins partiellement recyclées dans la cristallisation de masse fondue ou dans la distillation pour la séparation de composants de point d'ébullition faible.

6. Procédé selon la revendication 1 ou 2, **caractérisé en ce que** les cristaux de l'étape (c) ou de l'étape (d) sont introduits dans une distillation pour la séparation de l'eau.

7. Procédé selon la revendication 1 ou 2, **caractérisé en ce que** la liqueur mère après la séparation des cristaux à l'étape (c) et/ou la liqueur mère formée à l'étape (b) sont au moins partiellement introduites dans une séparation des composants de point d'ébullition élevé, lors de laquelle l'acide alcane-sulfonique est séparé par évaporation.

8. Procédé selon la revendication 7, **caractérisé en ce que** l'acide alcane-sulfonique séparé par évaporation est recyclé dans le processus chimique.

9. Procédé selon la revendication 8, **caractérisé en ce que** l'acide alcane-sulfonique séparé par évaporation est au moins partiellement condensé avant le recyclage dans le processus chimique.

10. Procédé selon la revendication 7, **caractérisé en ce que** la liqueur mère est partiellement condensée après l'élimination des composants de point d'ébullition élevé, et la fraction condensée est recyclée dans la distillation pour la séparation des composants de point d'ébullition faible ou dans la cristallisation de masse fondue de l'étape (b), et la fraction non condensée est évacuée du processus en tant que composant de point d'ébullition faible.

11. Procédé selon l'une quelconque des revendications 1 à 10, **caractérisé en ce que** l'acide alcane-sulfonique est l'acide méthane-sulfonique.

12. Procédé selon l'une quelconque des revendications 1 à 11, **caractérisé en ce que** le processus chimique est une alkylation ou une réaction d'estérification.

13. Procédé selon l'une quelconque des revendications 1 à 12, **caractérisé en ce que** le processus chimique est une alkylation d'acide salicylique ou de composés aromatiques substitués ou non substitués.

14. Procédé selon la revendication 13, **caractérisé en ce que** le processus chimique est la fabrication d'alkylbenzène linéaire à partir de benzène ou de benzène substitué avec une oléfine en présence d'un acide alcane-sulfonique en tant que catalyseur.

15. Procédé selon l'une quelconque des revendications 1 à 14, **caractérisé en ce que** la cristallisation de masse fondue est une cristallisation en suspension ou une cristallisation en couches.
